# EUROPEAN PATENT APPLICATION

(11) **EP 1 591 523 A1**
(43) Date of publication of application: **02.11.2005**
(21) Application number: 04707623.7
(22) Date of filing: 03.02.2004
(51) Int. Cl.: C12N 15/09, C12N 5/06

(54) **OVEREXPRESSION VECTOR FOR ANIMAL CELL**

(30) Priority: 03.02.2003 JP 2003025677
(71) Applicant: IMMUNO JAPAN INC., Tokyo 167-0051 (JP)
(72) Inventor: OKABE, Masato, Yonago-shi, Tottori 683-0064 (JP); NAKAMURA, Tetsuo, Tokyo 167-0051 (JP)
(74) Representative: Sternagel, Fleischer, Godemeyer & Partner
(86) International application number: PCT/JP2004/001066
(87) International publication number: WO 2004/070030

(57) **Abstract**

An object of the present invention is to provide an expression vector which enables easy obtainment of high-level productive strains, using a mammalian cell, and especially, a Chinese hamster ovary cell (CHO cell) as a host. The present invention provides an expression vector inducing high productivity of genetically recombinant proteins in animal host cells, which comprises a highly expression-inducible promoter, a multicloning site for insertion of gene and a polyadenylation signal sequence in this order from upstream, and further comprises downstream thereof a drug resistant gene that does not have a promoter capable of acting in animal cells.

SEQUENCE LISTING
<110> Immuno Japan Inc.
<120> High expression vector for animal cell
<130> A41056A
<160> 51

<210> 1
<211> 27
<212> DNA
<213> Artificial Sequence
<220>
<223> Description of Artificial Sequence: Synthetic DNA
<400> 1
tttccgcggt ttttccatag gctccgc 27

<210> 2
<211> 26
<212> DNA
<213> Artificial Sequence
<220>
<223> Description of Artificial Sequence: Synthetic DNA
<400> 2
tttatcgata ggatcttctt gagatc 26

<210> 3
<211> 29
<212> DNA

<213> Artificial Sequence

<220>

<223> Description of Artificial Sequence: Synthetic DNA

<400> 3

aagcttaaac gtacgaaaga attcaaaat                    29

<210> 4

<211> 35

<212> DNA

<213> Artificial Sequence

<220>

<223> Description of Artificial Sequence: Synthetic DNA

<400> 4

cgattttgaa ttctttcgta cgtttaagct tggcc             35

<210> 5

<211> 35

<212> DNA

<213> Artificial Sequence

<220>

<223> Description of Artificial Sequence: Synthetic DNA

<400> 5

ctgcagacca tgggctacca tggcgatagc tagac             35

<210> 6

<211> 20

<212> DNA

<213> Artificial Sequence

<220>

<223> Description of Artificial Sequence: Synthetic DNA

<400> 6

tcctgcagtt cattcagggc                                    20

<210> 7

<211> 28

<212> DNA

<213> Artificial Sequence

<220>

<223> Description of Artificial Sequence: Synthetic DNA

<400> 7

ttaagcttcg atgtacgggc cagatata                           28

<210> 8

<211> 39

<212> DNA

<213> Artificial Sequence

<220>

<223> Description of Artificial Sequence: Synthetic DNA

<400> 8

ttgcggccgc cccgtcgaca atttcgataa gccagttaa               39

<210> 9

<211> 36

<212> DNA

<213> Artificial Sequence

<220>

<223> Description of Artificial Sequence: Synthetic DNA

<400> 9

ttgcggccgc cttctagagc ctcgactgtg ccttct          36

<210> 10

<211> 25

<212> DNA

<213> Artificial Sequence

<220>

<223> Description of Artificial Sequence: Synthetic DNA

<400> 10

ttggatcctc cccagcatgc ctgct                      25

<210> 11

<211> 41

<212> DNA

<213> Artificial Sequence

<220>

<223> Description of Artificial Sequence: Synthetic DNA

<400> 11

aatttcgtac gaagcttaaa ggatccgaat tcaacgtacg t          41

<210> 12

<211> 41

<212> DNA

<213> Artificial Sequence

<220>

<223> Description of Artificial Sequence: Synthetic DNA

<400> 12

agctacgtac gttgaattcg gatcctttaa gcttcgtacg a 41

<210> 13

<211> 41

<212> DNA

<213> Artificial Sequence

<220>

<223> Description of Artificial Sequence: Synthetic DNA

<400> 13

tgcttaaagc ggccgcttat ctagaaccat gggctaccat g 41

<210> 14

<211> 27

<212> DNA

<213> Artificial Sequence

<220>

<223> Description of Artificial Sequence: Synthetic DNA

<400> 14

taagctagct cagaagaact cgtcaag 27

<210> 15

<211> 20

<212> DNA

<213> Artificial Sequence

<220>

<223> Description of Artificial Sequence: Synthetic DNA

<400> 15

ggtttatcgt tgtgacttta                                20

<210> 16

<211> 26

<212> DNA

<213> Artificial Sequence

<220>

<223> Description of Artificial Sequence: Synthetic DNA

<400> 16

agcttaaagt cacaacgata aaccgc                         26

<210> 17

<211> 30

<212> DNA

<213> Artificial Sequence

<220>

<223> Description of Artificial Sequence: Synthetic DNA

<400> 17

gcggccgctt ctagagcctc gactgtgcct                     30

<210> 18

<211> 25

<212> DNA

<213> Artificial Sequence

<220>

<223> Description of Artificial Sequence: Synthetic DNA

<400> 18

ttgctagctc cccagcatgc ctgct                    25

<210> 19

<211> 28

<212> DNA

<213> Artificial Sequence

<220>

<223> Description of Artificial Sequence: Synthetic DNA

<400> 19

tgctcgagga gaacttcagg gtgagttt                 28

<210> 20

<211> 29

<212> DNA

<213> Artificial Sequence

<220>

<223> Description of Artificial Sequence: Synthetic DNA

<400> 20

tggcggccgc cagcacgttg cccaggagc                29

<210> 21

<211> 5019

<212> DNA

<213> Artificial Sequence

<220>

<223> Description of Artificial Sequence: plasmid

<400> 23

gcggccgctt ctagagcctc gacdgtgcct tctagttgcc agccatctgt tgtttgcccc 60

tcccccgtgc cttccttgac cctggaaggt gccactccca ctgtcctttc ctaataaaat 120

gaggaaattg catcgcattg tctgagtagg tgtcattcta ttctgggggg tggggtgggg 180

caggacagca agggggagga ttgggaagac aatagcaggc atgctgggga gctagaacca 240

tgggctacca tggcgatagc tagactgggc ggttttatgg acagcaagcg aaccggaatt 300

gccagctggg gcgccctctg gtaaggttgg gaagccctgc aaagtaaact ggatggcttt 360

cttgccgcca aggatctgat ggcgcagggg atcaagatct gatcaagaga caggatgagg 420

atcgtttcgc atgattgaac aagatggatt gcacgcaggt tctccggccg cttgggtgga 480

gaggctattc ggctatgact gggcacaaca gacaatcggc tgctctgatg ccgccgtgtt 540

ccggctgtca gcgcaggggc gcccggttct ttttgtcaag accgacctgt ccggtgccct 600

gaatgaactg caggacgagg cagcgcggct atcgtggctg gccacgacgg gcgttccttg 660

cgcagctgtg ctcgacgttg tcactgaagc gggaagggac tggctgctat tgggcgaagt 720

gccggggcag gatctcctgt catctcacct tgctcctgcc gagaaagtat ccatcatggc 780

tgatgcaatg cggcggctgc atacgcttga tccggctacc tgcccattcg accaccaagc 840

gaaacatcgc atcgagcgag cacgtactcg gatggaagcc ggtcttgtcg atcaggatga 900

tctggacgaa gagcatcagg ggctcgcgcc agccgaactg ttcgccaggc tcaaggcgcg 960

catgcccgac ggcgaggatc tcgtcgtgac ccatggcgat gcctgcttgc cgaatatcat 1020

ggtggaaaat ggccgctttt ctggattcat cgactgtggc cggctgggtg tggcggaccg 1080

ctatcaggac atagcgttgg ctacccgtga tattgctgaa gagcttggcg gcgaatgggc 1140

tgaccgcttc ctcgtgcttt acggtatcgc cgctcccgat tcgcagcgca tcgccttcta 1200

tcgccttctt gacgagttct tctgaggatc tgcctcgact gtgccttcta gttgccagcc 1260

atctgttgtt tgcccctccc ccgtgccttc cttgaccctg gaaggtgcca ctcccactgt 1320

cctttcctaa taaaatgagg aaattgcatc gcattgtctg agtaggtgtc attctattct 1380

ggggggtggg gtggggcagg acagcaaggg ggaggattgg gaagacaata gcaggcatgc 1440
tggggaggat ccttgaattc atctcaatta gtcagcaacc atagtcccgc ccctaactcc 1500
gcccatcccg cccctaactc cgcccagttc cgcccattct ccgccccatg gctgactaat 1560
tttttttatt tatgcagagg ccgaggccgc ctcggcctct gagctattcc agaagtagtg 1620
aggaggcttt tttggaggcc taggcttttg caaaaagctc tgcagatggt tcgaccattg 1680
aactgcatcg tcgccgtgtc ccaaaatatg gggattggca agaacggaga cctaccctgg 1740
cctccgctca ggaacgagtt caagtacttc caaagaatga ccacaacctc ttcagtggaa 1800
ggtaaacaga atctggtgat tatgggtagg aaaacctggt tctccattcc tgagaagaat 1860
cgacctttaa aggacagaat taatatagtt ctcagtagag aactcaaaga accaccacga 1920
ggagctcatt ttcttgccaa aagtttggat gatgccttaa gacttattga acaaccggaa 1980
ttggcaagta aagtagacat ggtttggata gtcggaggca gttctgttta ccaggaagcc 2040
atgaatcaac caggccacct cagactcttt gtgacaagga tcatgcagga atttgaaagt 2100
gacacgtttt tcccagaaat tgatttgggg aaatataaac ttctcccaga atacccaggc 2160
gtcctctctg aggtccagga ggaaaaaggc atcaagtata agtttgaagt ctacgagaag 2220
aaagactaac aggaagatgc tttcaagttc tctgctcccc tcctaaagct atgcattttt 2280
ataagaccat gggacttttg ctggctttag atccgtgaca taattggaca aactacctac 2340
agagatttaa agctctaagg taaatataaa atttttaagt gtataatgtg ttaaactact 2400
gattctaatt gtttgtgtat tttagattcc aacctatgga actgatgaat gggagcagtg 2460
gtggaatgcc tttaatgagg aaaacctgtt ttgctcagaa gaaatgccat ctagtgatga 2520
tgaggctact gctgactctc aacattctac tcctccaaaa aagaagagaa aggtagaaga 2580
ccccaaggac tttccttcag aattgctaag ttttttgagt catgctgtgt ttagtaatag 2640
aactcttgct tgctttgcta tttacaccac aaaggaaaaa gctgcactgc tatacaagaa 2700
aattatggaa aaatattctg taacctttat aagtaggcat aacagttata atcataacat 2760
actgtttttt cttactccac acaggcatag agtgtctgct attaataact atgctcaaaa 2820
attgtgtacc tttagctttt taatttgtaa aggggttaat aaggaatatt tgatgtatag 2880
tgccttgact agagatcata atcagccata ccacatttgt agaggtttta cttgctttaa 2940
aaaacctccc acacctcccc ctgaacctga aacataaaat gaatgcaatt gttgttgtta 3000

acttgtttat tgcagcttat aatggttaca aataaagcaa tagcatcaca aatttcacaa 3060
ataaagcatt tttttcactg cattctagtt gtggtttgtc caaactcatc aatgtatctt 3120
atcatgtctg ggcccatcga taggatcttc ttgagatcct ttttttctgc gcgtaatctg 3180
ctgcttgcaa acaaaaaaac caccgctacc agcggtggtt tgtttgccgg atcaagagct 3240
accaactctt tttccgaagg taactggctt cagcagagcg cagataccaa atactgttct 3300
tctagtgtag ccgtagttag gccaccactt caagaactct gtagcaccgc ctacatacct 3360
cgctctgcta atcctgttac cagtggctgc tgccagtggc gataagtcgt gtcttaccgg 3420
gttggactca agacgatagt taccggataa ggcgcagcgg tcgggctgaa cggggggttc 3480
gtgcacacag cccagcttgg agcgaacgac ctacaccgaa ctgagatacc tacagcgtga 3540
gctatgagaa agcgccacgc ttcccgaagg gagaaaggcg gacaggtatc cggtaagcgg 3600
cagggtcgga acaggagagc gcacgaggga gcttccaggg ggaaacgcct ggtatcttta 3660
tagtcctgtc gggtttcgcc acctctgact tgagcgtcga tttttgtgat gctcgtcagg 3720
ggggcggagc ctatggaaaa accgcggtta agcttcgatg tacgggccag atatacgcgt 3780
tgacattgat tattgactag ttattaatag taatcaatta cggggtcatt agttcatagc 3840
ccatatatgg agttccgcgt tacataactt acggtaaatg gcccgcctgg ctgaccgccc 3900
aacgaccccc gcccattgac gtcaataatg acgtatgttc ccatagtaac gccaataggg 3960
actttccatt gacgtcaatg ggtggagtat ttacggtaaa ctgcccactt ggcagtacat 4020
caagtgtatc atatgccaag tacgccccct attgacgtca atgacggtaa atggcccgcc 4080
tggcattatg cccagtacat gaccttatgg gactttccta cttggcagta catctacgta 4140
ttagtcatcg ctattaccat ggtgatgcgg ttttggcagt acatcaatgg gcgtggatag 4200
cggtttgact cacggggatt tccaagtctc caccccattg acgtcaatgg gagtttgttt 4260
tggcaccaaa atcaacggga ctttccaaaa tgtcgtaaca actccgcccc attgacgcaa 4320
atgggcggta ggcgtgtacg gtgggaggtc tatataagca gagctctctg gctaactaga 4380
gaacccactg ttaactggct tatcgaaatt gtcgaggaga acttcagggt gagtttgggg 4440
acccttgatt gttctttctt tttcgctatt gtaaaattca tgttatatgg agggggcaaa 4500
gttttcaggg tgttgtttag aatgggaaga tgtcccttgt atcaccatgg accctcatga 4560
taattttgtt tctttcactt tctactctgt tgacaaccat tgtctcctct tattttcttt 4620

tcattttctg taactttttc gttaaacttt agcttgcatt tgtaacgaat ttttaaattc 4680
acttttgttt atttgtcaga ttgtaagtac tttctctaat cacttttttt tcaaggcaat 4740
cagggtatat tatattgtac ttcagcacag ttttagagaa caattgttat aattaaatga 4800
taaggtagaa tatttctgca tataaattct ggctggcgtg gaaatattct tattggtaga 4860
aacaactaca tcctggtcat catcctgcct ttctctttat ggttacaatg atatacactg 4920
tttgagatga ggataaaata ctctgagtcc aaaccgggcc cctctgctaa ccatgttcat 4980
gccttcttct ttttcctaca gctcctgggc aacgtgctg 5019

<210> 22
<211> 33
<212> DNA
<213> Artificial Sequence
<220>
<223> Description of Artificial Sequence: Synthetic DNA
<400> 22
tagcggccgc accatggctg gacctgccac cca 33

<210> 23
<211> 28
<212> DNA
<213> Artificial Sequence
<220>
<223> Description of Artificial Sequence: Synthetic DNA
<400> 23
tatctagatc agggctgggc aaggtggcg 28

<210> 24

<211> 5666

<212> DNA

<213> Artificial Sequence

<220>

<223> Description of Artificial Sequence: plasmid

<400> 24

gcggccgcac catggctgga cctgccaccc agagccccat gaagctgatg gccctgcagc 60

tgctgctgtg gcacagtgca ctctggacag tgcaggaagc cacccccctg ggccctgcca 120

gctccctgcc ccagagcttc ctgctcaagt gcttagagca agtgaggaag atccagggcg 180

atggcgcagc gctccaggag aagctgtgtg ccacctacaa gctgtgccac cccgaggagc 240

tggtgctgct cggacactct ctgggcatcc cctgggctcc cctgagcagc tgccccagcc 300

aggccctgca gctggcaggc tgcttgagcc aactccatag cggccttttc ctctaccagg 360

ggctcctgca ggccctggaa gggatctccc ccgagttggg tcccaccttg gacacactgc 420

agctggacgt cgccgacttt gccaccacca tctggcagca gatggaagaa ctgggaatgg 480

cccctgccct gcagcccacc cagggtgcca tgccggcctt cgcctctgct ttccagcgcc 540

gggcaggagg ggtcctagtt gcctcccatc tgcagagctt cctggaggtg tcgtaccgcg 600

ttctacgcca ccttgcccag ccctgatcta gataaatcca tatgactagt agatcctcta 660

gagcctcgac tgtgccttct agttgccagc catctgttgt ttgcccctcc cccgtgcctt 720

ccttgaccct ggaaggtgcc actcccactg tcctttccta ataaaatgag gaaattgcat 780

cgcattgtct gagtaggtgt cattctattc tggggggtgg ggtggggcag gacagcaagg 840

gggaggattg ggaagacaat agcaggcatg ctggggagct agaaccatgg gctaccatgg 900

cgatagctag actgggcggt tttatggaca gcaagcgaac cggaattgcc agctggggcg 960

ccctctggta aggttgggaa gccctgcaaa gtaaactgga tggctttctt gccgccaagg 1020

atctgatggc gcaggggatc aagatctgat caagagacag gatgaggatc gtttcgcatg 1080

attgaacaag atggattgca cgcaggttct ccggccgctt gggtggagag gctattcggc 1140

tatgactggg cacaacagac aatcggctgc tctgatgccg ccgtgttccg gctgtcagcg 1200

caggggcgcc cggttctttt tgtcaagacc gacctgtccg gtgccctgaa tgaactgcag 1260

gacgaggcag cgcggctatc gtggctggcc acgacgggcg ttccttgcgc agctgtgctc 1320
gacgttgtca ctgaagcggg aagggactgg ctgctattgg gcgaagtgcc ggggcaggat 1380
ctcctgtcat ctcaccttgc tcctgccgag aaagtatcca tcatggctga tgcaatgcgg 1440
cggctgcata cgcttgatcc ggctacctgc ccattcgacc accaagcgaa acatcgcatc 1500
gagcgagcac gtactcggat ggaagccggt cttgtcgatc aggatgatct ggacgaagag 1560
catcaggggc tcgcgccagc cgaactgttc gccaggctca aggcgcgcat gcccgacggc 1620
gaggatctcg tcgtgaccca tggcgatgcc tgcttgccga atatcatggt ggaaaatggc 1680
cgcttttctg gattcatcga ctgtggccgg ctgggtgtgg cggaccgcta tcaggacata 1740
gcgttggcta cccgtgatat tgctgaagag cttggcggcg aatgggctga ccgcttcctc 1800
gtgctttacg gtatcgccgc tcccgattcg cagcgcatcg ccttctatcg ccttcttgac 1860
gagttcttct gaggatctgc ctcgactgtg ccttctagtt gccagccatc tgttgtttgc 1920
ccctcccccg tgccttcctt gaccctggaa ggtgccactc ccactgtcct ttcctaataa 1980
aatgaggaaa ttgcatcgca ttgtctgagt aggtgtcatt ctattctggg gggtggggtg 2040
gggcaggaca gcaaggggga ggattgggaa gacaatagca ggcatgctgg ggaggatcct 2100
tgaattcatc tcaattagtc agcaaccata gtcccgcccc taactccgcc catcccgccc 2160
ctaactccgc ccagttccgc ccattctccg ccccatggct gactaatttt ttttatttat 2220
gcagaggccg aggccgcctc ggcctctgag ctattccaga agtagtgagg aggctttttt 2280
ggaggcctag gcttttgcaa aaagctctgc agatggttcg accattgaac tgcatcgtcg 2340
ccgtgtccca aaatatgggg attggcaaga acggagacct accctggcct ccgctcagga 2400
acgagttcaa gtacttccaa agaatgacca caacctcttc agtggaaggt aaacagaatc 2460
tggtgattat gggtaggaaa acctggttct ccattcctga gaagaatcga cctttaaagg 2520
acagaattaa tatagttctc agtagagaac tcaaagaacc accacgagga gctcattttc 2580
ttgccaaaag tttggatgat gccttaagac ttattgaaca accggaattg gcaagtaaag 2640
tagacatggt ttggatagtc ggaggcagtt ctgtttacca ggaagccatg aatcaaccag 2700
gccacctcag actctttgtg acaaggatca tgcaggaatt tgaaagtgac acgtttttcc 2760
cagaaattga tttggggaaa tataaacttc tcccagaata cccaggcgtc ctctctgagg 2820
tccaggagga aaaaggcatc aagtataagt ttgaagtcta cgagaagaaa gactaacagg 2880

aagatgcttt caagttctct gctcccctcc taaagctatg catttttata agaccatggg 2940
acttttgctg gctttagatc cgtgacataa ttggacaaac tacctacaga gatttaaagc 3000
tctaaggtaa atataaaatt tttaagtgta taatgtgtta aactactgat tctaattgtt 3060
tgtgtatttt agattccaac ctatggaact gatgaatggg agcagtggtg gaatgccttt 3120
aatgaggaaa acctgttttg ctcagaagaa atgccatcta gtgatgatga ggctactgct 3180
gactctcaac attctactcc tccaaaaaag aagagaaagg tagaagaccc caaggacttt 3240
ccttcagaat tgctaagttt tttgagtcat gctgtgttta gtaatagaac tcttgcttgc 3300
tttgctattt acaccacaaa ggaaaaagct gcactgctat acaagaaaat tatggaaaaa 3360
tattctgtaa cctttataag taggcataac agttataatc ataacatact gttttttctt 3420
actccacaca ggcatagagt gtctgctatt aataactatg ctcaaaaatt gtgtaccttt 3480
agctttttaa tttgtaaagg ggttaataag gaatatttga tgtatagtgc cttgactaga 3540
gatcataatc agccatacca catttgtaga ggttttactt gctttaaaaa acctcccaca 3600
cctccccctg aacctgaaac ataaaatgaa tgcaattgtt gttgttaact tgtttattgc 3660
agcttataat ggttacaaat aaagcaatag catcacaaat ttcacaaata aagcattttt 3720
ttcactgcat tctagttgtg gtttgtccaa actcatcaat gtatcttatc atgtctgggc 3780
ccatcgatag gatcttcttg agatcctttt tttctgcgcg taatctgctg cttgcaaaca 3840
aaaaaaccac cgctaccagc ggtggtttgt ttgccggatc aagagctacc aactcttttt 3900
ccgaaggtaa ctggcttcag cagagcgcag ataccaaata ctgttcttct agtgtagccg 3960
tagttaggcc accacttcaa gaactctgta gcaccgccta catacctcgc tctgctaatc 4020
ctgttaccag tggctgctgc cagtggcgat aagtcgtgtc ttaccgggtt ggactcaaga 4080
cgatagttac cggataaggc gcagcggtcg ggctgaacgg ggggttcgtg cacacagccc 4140
agcttggagc gaacgaccta caccgaactg agatacctac agcgtgagct atgagaaagc 4200
gccacgcttc ccgaagggag aaaggcggac aggtatccgg taagcggcag ggtcggaaca 4260
ggagagcgca cgagggagct tccaggggga aacgcctggt atctttatag tcctgtcggg 4320
tttcgccacc tctgacttga gcgtcgattt ttgtgatgct cgtcaggggg gcggagccta 4380
tggaaaaacc gcggttaagc ttcgatgtac gggccagata tacgcgttga cattgattat 4440
tgactagtta ttaatagtaa tcaattacgg ggtcattagt tcatagccca tatatggagt 4500

tccgcgttac ataacttacg gtaaatggcc cgcctggctg accgcccaac gacccccgcc 4560
cattgacgtc aataatgacg tatgttccca tagtaacgcc aatagggact ttccattgac 4620
gtcaatgggt ggagtattta cggtaaactg cccacttggc agtacatcaa gtgtatcata 4680
tgccaagtac gccccctatt gacgtcaatg acggtaaatg gcccgcctgg cattatgccc 4740
agtacatgac cttatgggac tttcctactt ggcagtacat ctacgtatta gtcatcgcta 4800
ttaccatggt gatgcggttt tggcagtaca tcaatgggcg tggatagcgg tttgactcac 4860
ggggatttcc aagtctccac cccattgacg tcaatgggag tttgttttgg caccaaaatc 4920
aacgggactt tccaaaatgt cgtaacaact ccgccccatt gacgcaaatg ggcggtaggc 4980
gtgtacggtg ggaggtctat ataagcagag ctctctggct aactagagaa cccactgtta 5040
actggcttat cgaaattgtc gaggagaact tcagggtgag tttggggacc cttgattgtt 5100
ctttcttttt cgctattgta aaattcatgt tatatggagg gggcaaagtt ttcagggtgt 5160
tgtttagaat gggaagatgt cccttgtatc accatggacc ctcatgataa ttttgtttct 5220
ttcactttct actctgttga caaccattgt ctcctcttat tttcttttca ttttctgtaa 5280
ctttttcgtt aaactttagc ttgcatttgt aacgaatttt taaattcact tttgtttatt 5340
tgtcagattg taagtacttt ctctaatcac ttttttttca aggcaatcag ggtatattat 5400
attgtacttc agcacagttt tagagaacaa ttgttataat taaatgataa ggtagaatat 5460
ttctgcatat aaattctggc tggcgtggaa atattcttat tggtagaaac aactacatcc 5520
tggtcatcat cctgcctttc tctttatggt tacaatgata tacactgttt gagatgagga 5580
taaaatactc tgagtccaaa ccgggcccct ctgctaacca tgttcatgcc ttcttctttt 5640
tcctacagct cctgggcaac gtgctg 5666

<210> 25
<211> 5648
<212> DNA
<213> Artificial Sequence
<220>
<223> Description of Artificial Sequence: plasmid

<400> 25

gcggccgcac catggctgga cctgccaccc agagccccat gaagctgatg gccctgcagc 60

tgctgctgtg gcacagtgca ctctggacag tgcaggaagc cacccccctg ggccctgcca 120

gctccctgcc ccagagcttc ctgctcaagt gcttagagca agtgaggaag atccagggcg 180

atggcgcagc gctccaggag aagctgtgtg ccacctacaa gctgtgccac cccgaggagc 240

tggtgctgct cggacactct ctgggcatcc cctgggctcc cctgagcagc tgccccagcc 300

aggccctgca gctggcaggc tgcttgagcc aactccatag cggccttttc ctctaccagg 360

ggctcctgca ggccctggaa gggatctccc ccgagttggg tcccaccttg gacacactgc 420

agctggacgt cgccgacttt gccaccacca tctggcagca gatggaagaa ctgggaatgg 480

cccctgccct gcagcccacc cagggtgcca tgccggcctt cgcctctgct ttccagcgcc 540

gggcaggagg ggtcctagtt gcctcccatc tgcagagctt cctggaggtg tcgtaccgcg 600

ttctacgcca ccttgcccag ccctgatcta gataaatcca tatgactagt agatcctcta 660

gactgcccgg gtggcatccc tgtgacccct ccccagtgcc tctcctggtc gtggaaggtg 720

ctactccagt gcccaccagc cttgtcctaa taaaattaag ttgcatcatt ttgtttgact 780

aggtgtcctt gtataatatt atggggtgga ggcgggtggt atggagcaag gggcaggttg 840

ggaagacaac ctgtagggcc tgcggggtgc tagagcagtg ggcttacatg gcgatagcta 900

gactgggcgg ttttatggac agcaagcgaa ccggaattgc cagctggggc gccctctggt 960

aaggttggga agccctgcaa agtaaactgg atggctttct tgccgccaag gatctgatgg 1020

cgcaggggat caagatctga tcaagagaca ggatgaggat cgtttcgcat gattgaacaa 1080

gatggattgc acgcaggttc tccggccgct tgggtggaga ggctattcgg ctatgactgg 1140

gcacaacaga caatcggctg ctctgatgcc gccgtgttcc ggctgtcagc gcaggggcgc 1200

ccggttcttt ttgtcaagac cgacctgtcc ggtgccctga atgaactgca ggacgaggca 1260

gcgcggctat cgtggctggc cacgacgggc gttccttgcg cagctgtgct cgacgttgtc 1320

actgaagcgg gaagggactg gctgctattg ggcgaagtgc cggggcagga tctcctgtca 1380

tctcaccttg ctcctgccga gaaagtatcc atcatggctg atgcaatgcg gcggctgcat 1440

acgcttgatc cggctacctg cccattcgac caccaagcga aacatcgcat cgagcgagca 1500

cgtactcgga tggaagccgg tcttgtcgat caggatgatc tggacgaaga gcatcagggg 1560

ctcgcgccag ccgaactgtt cgccaggctc aaggcgcgca tgcccgacgg cgaggatctc 1620
gtcgtgaccc atggcgatgc ctgcttgccg aatatcatgg tggaaaatgg ccgcttttct 1680
ggattcatcg actgtggccg gctgggtgtg gcggaccgct atcaggacat agcgttggct 1740
acccgtgata ttgctgaaga gcttggcggc gaatgggctg accgcttcct cgtgctttac 1800
ggtatcgccg ctcccgattc gcagcgcatc gccttctatc gccttcttga cgagttcttc 1860
tgaggatctc tgcccgggtg gcatccctgt gacccctccc cagtgcctct cctggtcgtg 1920
gaaggtgcta ctccagtgcc caccagcctt gtcctaataa aattaagttg catcattttg 1980
tttgactagg tgtccttgta taatattatg gggtggaggc gggtggtatg gagcaagggg 2040
caggttggga agacaacctg tagggcctgc ggggtggatc cttgaattca tctcaattag 2100
tcagcaacca tagtcccgcc cctaactccg cccatcccgc ccctaactcc gcccagttcc 2160
gcccattctc cgccccatgg ctgactaatt ttttttattt atgcagaggc cgaggccgcc 2220
tcggcctctg agctattcca gaagtagtga ggaggctttt ttggaggcct aggcttttgc 2280
aaaaagctct gcagatggtt cgaccattga actgcatcgt cgccgtgtcc caaaatatgg 2340
ggattggcaa gaacggagac ctaccctggc ctccgctcag gaacgagttc aagtacttcc 2400
aaagaatgac cacaacctct tcagtggaag gtaaacagaa tctggtgatt atgggtagga 2460
aaacctggtt ctccattcct gagaagaatc gacctttaaa ggacagaatt aatatagttc 2520
tcagtagaga actcaaagaa ccaccacgag gagctcattt tcttgccaaa agtttggatg 2580
atgccttaag acttattgaa caaccggaat tggcaagtaa agtagacatg gtttggatag 2640
tcggaggcag ttctgtttac caggaagcca tgaatcaacc aggccacctc agactctttg 2700
tgacaaggat catgcaggaa tttgaaagtg acacgttttt cccagaaatt gatttgggga 2760
aatataaact tctcccagaa tacccaggcg tcctctctga ggtccaggag gaaaaaggca 2820
tcaagtataa gtttgaagtc tacgagaaga aagactaaca ggaagatgct ttcaagttct 2880
ctgctcccct cctaaagcta tgcattttta taagaccatg ggacttttgc tggctttaga 2940
tccgtgacat aattggacaa actacctaca gagatttaaa gctctaaggt aaatataaaa 3000
tttttaagtg tataatgtgt taaactactg attctaattg tttgtgtatt ttagattcca 3060
acctatggaa ctgatgaatg ggagcagtgg tggaatgcct ttaatgagga aaacctgttt 3120
tgctcagaag aaatgccatc tagtgatgat gaggctactg ctgactctca acattctact 3180

cctccaaaaa agaagagaaa ggtagaagac cccaaggact ttccttcaga attgctaagt 3240
tttttgagtc atgctgtgtt tagtaataga actcttgctt gctttgctat ttacaccaca 3300
aaggaaaaag ctgcactgct atacaagaaa attatggaaa aatattctgt aacctttata 3360
agtaggcata acagttataa tcataacata ctgttttttc ttactccaca caggcataga 3420
gtgtctgcta ttaataacta tgctcaaaaa ttgtgtacct ttagcttttt aatttgtaaa 3480
ggggttaata aggaatattt gatgtatagt gccttgacta gagatcataa tcagccatac 3540
cacatttgta gaggttttac ttgctttaaa aaacctccca cacctccccc tgaacctgaa 3600
acataaaatg aatgcaattg ttgttgttaa cttgtttatt gcagcttata atggttacaa 3660
ataaagcaat agcatcacaa atttcacaaa taaagcattt ttttcactgc attctagttg 3720
tggtttgtcc aaactcatca atgtatctta tcatgtctgg gcccatcgat aggatcttct 3780
tgagatcctt tttttctgcg cgtaatctgc tgcttgcaaa caaaaaaacc accgctacca 3840
gcggtggttt gtttgccgga tcaagagcta ccaactcttt ttccgaaggt aactggcttc 3900
agcagagcgc agataccaaa tactgttctt ctagtgtagc cgtagttagg ccaccacttc 3960
aagaactctg tagcaccgcc tacatacctc gctctgctaa tcctgttacc agtggctgct 4020
gccagtggcg ataagtcgtg tcttaccggg ttggactcaa gacgatagtt accggataag 4080
gcgcagcggt cgggctgaac ggggggttcg tgcacacagc ccagcttgga gcgaacgacc 4140
tacaccgaac tgagatacct acagcgtgag ctatgagaaa gcgccacgct tcccgaaggg 4200
agaaaggcgg acaggtatcc ggtaagcggc agggtcggaa caggagagcg cacgagggag 4260
cttccagggg gaaacgcctg gtatctttat agtcctgtcg ggtttcgcca cctctgactt 4320
gagcgtcgat ttttgtgatg ctcgtcaggg gggcggagcc tatggaaaaa ccgcggttaa 4380
gcttcgatgt acgggccaga tatacgcgtt gacattgatt attgactagt tattaatagt 4440
aatcaattac ggggtcatta gttcatagcc catatatgga gttccgcgtt acataactta 4500
cggtaaatgg cccgcctggc tgaccgccca acgacccccg cccattgacg tcaataatga 4560
cgtatgttcc catagtaacg ccaataggga ctttccattg acgtcaatgg gtggagtatt 4620
tacggtaaac tgcccacttg gcagtacatc aagtgtatca tatgccaagt acgcccccta 4680
ttgacgtcaa tgacggtaaa tggcccgcct ggcattatgc ccagtacatg accttatggg 4740
actttcctac ttggcagtac atctacgtat tagtcatcgc tattaccatg gtgatgcggt 4800

tttggcagta catcaatggg cgtggatagc ggtttgactc acggggattt ccaagtctcc 4860
accccattga cgtcaatggg agtttgtttt ggcaccaaaa tcaacgggac tttccaaaat 4920
gtcgtaacaa ctccgcccca ttgacgcaaa tgggcggtag gcgtgtacgg tgggaggtct 4980
atataagcag agctctctgg ctaactagag aacccactgt taactggctt atcgaaattg 5040
tcgaggagaa cttcagggtg agtttgggga cccttgattg ttctttcttt ttcgctattg 5100
taaaattcat gttatatgga gggggcaaag ttttcagggt gttgtttaga atgggaagat 5160
gtcccttgta tcaccatgga ccctcatgat aattttgttt ctttcacttt ctactctgtt 5220
gacaaccatt gtctcctctt attttctttt cattttctgt aactttttcg ttaaacttta 5280
gcttgcattt gtaacgaatt tttaaattca cttttgttta tttgtcagat tgtaagtact 5340
ttctctaatc actttttttt caaggcaatc agggtatatt atattgtact tcagcacagt 5400
tttagagaac aattgttata attaaatgat aaggtagaat atttctgcat ataaattctg 5460
gctggcgtgg aaatattctt attggtagaa acaactacat cctggtcatc atcctgcctt 5520
tctctttatg gttacaatga tatacactgt ttgagatgag gataaaatac tctgagtcca 5580
aaccgggccc ctctgctaac catgttcatg ccttcttctt tttcctacag ctcctgggca 5640
acgtgctg 5648

<210> 26
<211> 30
<212> DNA
<213> Artificial Sequence
<220>
<223> Description of Artificial Sequence: Synthetic DNA
<400> 26
ggatcctcac tctcttccgc atcgctgtct 30

<210> 27
<211> 43

<212> DNA

<213> Artificial Sequence

<220>

<223> Description of Artificial Sequence: Synthetic DNA

<400> 27

agagtttgtc ctcaaccgcg agcccaacag ctggccctcg cag          43

<210> 28

<211> 43

<212> DNA

<213> Artificial Sequence

<220>

<223> Description of Artificial Sequence: Synthetic DNA

<400> 28

ctgcgagggc cagctgttgg gctcgcggtt gaggacaaac tct          43

<210> 29

<211> 40

<212> DNA

<213> Artificial Sequence

<220>

<223> Description of Artificial Sequence: Synthetic DNA

<400> 29

tccctcggtg gcggagtacc gttcggaggc cgacgggttt          40

<210> 30

<211> 40

<212> DNA

<213> Artificial Sequence

<220>

<223> Description of Artificial Sequence: Synthetic DNA

<400> 30

aaacccgtcg gcctccgaac ggtactccgc caccgaggga                    40

<210> 31

<211> 33

<212> DNA

<213> Artificial Sequence

<220>

<223> Description of Artificial Sequence: Synthetic DNA

<400> 31

ccacacctca cctcgaccat ccgccgtctc aag                           33

<210> 32

<211> 33

<212> DNA

<213> Artificial Sequence

<220>

<223> Description of Artificial Sequence: Synthetic DNA

<400> 32

caggcttgag atccagctgt tggggtgagt act                           33

<210> 33

<211> 35

<212> DNA

<213> Artificial Sequence

<220>

<223> Description of Artificial Sequence: Synthetic DNA

<400> 33

ctcaagtgta tggccagatc gggccaggtg aatat                    35

<210> 34

<211> 30

<212> DNA

<213> Artificial Sequence

<220>

<223> Description of Artificial Sequence: Synthetic DNA

<400> 34

aagcttcgcg ttacataact tacggtaaat                          30

<210> 35

<211> 32

<212> DNA

<213> Artificial Sequence

<220>

<223> Description of Artificial Sequence: Synthetic DNA

<400> 35

agatctgacg gttcactaaa cgagctctgc tt                       32

<210> 36

<211> 45

<212> DNA

<213> Artificial Sequence

<220>

<223> Description of Artificial Sequence: Synthetic DNA

<400> 36

aaaggcaaag tggatgtcat tgtcactcaa gtgtatggcc agatc          45

<210> 37

<211> 45

<212> DNA

<213> Artificial Sequence

<220>

<223> Description of Artificial Sequence: Synthetic DNA

<400> 37

ctgggagtgg acacctgtgg agagaaaggc aaagtggatg tcatt          45

<210> 38

<211> 27

<212> DNA

<213> Artificial Sequence

<220>

<223> Description of Artificial Sequence: Synthetic DNA

<400> 38

gatctcaagc ctgccacacc tcacctc                              27

<210> 39

<211> 27

<212> DNA

<213> Artificial Sequence

<220>

<223> Description of Artificial Sequence: Synthetic DNA

<400> 39

gaggtgaggt gtggcaggct tgagatc 27

<210> 40

<211> 34

<212> DNA

<213> Artificial Sequence

<220>

<223> Description of Artificial Sequence: Synthetic DNA

<400> 40

gcggccgctt ggacctggga gtggacacct gtgg 34

<210> 41

<211> 538

<212> DNA

<213> Artificial Sequence

<220>

<223> Description of Artificial Sequence: CMV promoter

<400> 41

aagcttcgcg ttacataact tacggtaaat ggcccgcctg gctgaccgcc caacgacccc 60

cgcccattga cgtcaataat gacgtatgtt cccatagtaa cgccaatagg gactttccat 120

tgacgtcaat gggtggagta tttacggtaa actgcccact tggcagtaca tcaagtgtat 180

catatgccaa gtccgccccc tattgacgtc aatgacggta aatggcccgc ctggcattat 240

gcccagtaca tgaccttacg ggactttcct acttggcagt acatctacgt attagtcatc 300

gctattacca tggtgatgcg gttttggcag tacaccaatg ggcgtggata gcggtttgac 360

tcacggggat ttccaagtct ccaccccatt gacgtcaatg ggagtttgtt ttggcaccaa 420

aatcaacggg actttccaaa atgtcgtaat aaccccgccc cgttgacgca aatgggcggt 480

aggcgtgtac ggtgggaggt ctatataagc agagctcgtt tagtgaaccg tcagatct 538

<210> 42

<211> 335

<212> DNA

<213> Artificial Sequence

<220>

<223> Description of Artificial Sequence: mRNA

<400> 42

ggatcctcac tctcttccgc atcgctgtct gcgagggcca gctgttgggc tcgcggttga 60

ggacaaactc ttcgcggtct ttccagtact cttggatcgg aaacccgtcg gcctccgaac 120

ggtactccgc caccgaggga cctgagcgag tccgcatcga ccggatcgga aaacctctcg 180

agaaaggcgt ctaaccagtc acagtcgcaa ggtaggctga gcaccgtggc gggcggcagc 240

gggtggcggt cggggttgtt tctggcggag gtgctgctga tgatgtaatt aaagtaggcg 300

gtcttgagac ggcggatggt cgaggtgagg tgtgg 335

<210> 43

<211> 182

<212> DNA

<213> Artificial Sequence

<220>

<223> Description of Artificial Sequence: mRNA

<400> 43

caggcttgag atccagctgt tggggtgagt actccctctc aaaagcgggc atgacttctg 60
cgctaagatt gtcagtttcc aaaaacgagg aggatttgat attcacctgg cccgatctgg 120
ccatacactt gagtgacaat gacatccact ttgcctttct ctccacaggt gtccactccc 180
ag 182

<210> 44
<211> 348
<212> DNA
<213> Artificial Sequence
<220>
<223> Description of Artificial Sequence: DNA
<400> 44
ggatcctcac tctcttccgc atcgctgtct gcgagggcca gctgttgggc tcgcggttga 60
ggacaaactc ttcgcggtct ttccagtact cttggatcgg aaacccgtcg gcctccgaac 120
ggtactccgc caccgaggga cctgagcgag tccgcatcga ccggatcgga aaacctctcg 180
agaaaggcgt ctaaccagtc acagtcgcaa ggtaggctga gcaccgtggc gggcggcagc 240
gggtggcggt cggggttgtt tctggcggag gtgctgctga tgatgtaatt aaagtaggcg 300
gtcttgagac ggcggatggt cgaggtgagg tgtggcaggc ttgagatc 348

<210> 45
<211> 210
<212> DNA
<213> Artificial Sequence
<220>
<223> Description of Artificial Sequence: DNA
<400> 45
gaggtgaggt gtggcaggct tgagatccag ctgttggggt gagtactccc tctcaaaagc 60

gggcatgact tctgcgctaa gattgtcagt ttccaaaaac gaggaggatt tgatattcac 120
ctggcccgat ctggccatac acttgagtga caatgacatc cactttgcct ttctctccac 180
aggtgtccac tcccaggtcc aagcggccgc 210

<210> 46
<211> 531
<212> DNA
<213> Artificial Sequence
<220>
<223> Description of Artificial Sequence: DNA
<400> 46
ggatcctcac tctcttccgc atcgctgtct gcgagggcca gctgttgggc tcgcggttga 60
ggacaaactc ttcgcggtct ttccagtact cttggatcgg aaacccgtcg gcctccgaac 120
ggtactccgc caccgaggga cctgagcgag tccgcatcga ccggatcgga aaacctctcg 180
agaaaggcgt ctaaccagtc acagtcgcaa ggtaggctga gcaccgtggc gggcggcagc 240
gggtggcggt cggggttgtt tctggcggag gtgctgctga tgatgtaatt aaagtaggcg 300
gtcttgagac ggcggatggt cgaggtgagg tgtggcaggc ttgagatcca gctgttgggg 360
tgagtactcc ctctcaaaag cgggcatgac ttctgcgcta agattgtcag tttccaaaaa 420
cgaggaggat ttgatattca cctggcccga tctggccata cacttgagtg acaatgacat 480
ccactttgcc tttctctcca caggtgtcca ctcccaggtc caagcggccg c 531

<210> 47
<211> 3953
<212> DNA
<213> Artificial Sequence
<220>
<223> Description of Artificial Sequence: DNA

<400> 47

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgccga atttcgtacg aagcttcgcg 420

ttacataact tacggtaaat ggcccgcctg gctgaccgcc caacgacccc cgcccattga 480

cgtcaataat gacgtatgtt cccatagtaa cgccaatagg gactttccat tgacgtcaat 540

gggtggagta tttacggtaa actgcccact tggcagtaca tcaagtgtat catatgccaa 600

gtccgccccc tattgacgtc aatgacggta aatggcccgc ctggcattat gcccagtaca 660

tgaccttacg ggactttcct acttggcagt acatctacgt attagtcatc gctattacca 720

tggtgatgcg gttttggcag tacaccaatg ggcgtggata gcggtttgac tcacggggat 780

ttccaagtct ccaccccatt gacgtcaatg ggagtttgtt ttggcaccaa aatcaacggg 840

actttccaaa atgtcgtaat aaccccgccc cgttgacgca aatgggcggt aggcgtgtac 900

ggtgggaggt ctatataagc agagctcgtt tagtgaaccg tcagatcctc actctcttcc 960

gcatcgctgt ctgcgagggc cagctgttgg gctcgcggtt gaggacaaac tcttcgcggt 1020

ctttccagta ctcttggatc ggaaacccgt cggcctccga acggtactcc gccaccgagg 1080

gacctgagcg agtccgcatc gaccggatcg gaaaacctct cgagaaaggc gtctaaccag 1140

tcacagtcgc aaggtaggct gagcaccgtg gcgggcggca gcgggtggcg gtcggggttg 1200

tttctggcgg aggtgctgct gatgatgtaa ttaaagtagg cggtcttgag acggcggatg 1260

gtcgaggtga ggtgtggcag gcttgagatc cagctgttgg ggtgagtact ccctctcaaa 1320

agcgggcatg acttctgcgc taagattgtc agtttccaaa aacgaggagg atttgatatt 1380

cacctggccc gatctggcca tacacttgag tgacaatgac atccactttg cctttctctc 1440

cacaggtgtc cactcccagg tccaagcggc cgccttctag agcctcgact gtgccttcta 1500

gttgccagcc atctgttgtt tgcccctccc ccgtgccttc cttgaccctg gaaggtgcca 1560

ctcccactgt cctttcctaa taaaatgagg aaattgcatc gcattgtctg agtaggtgtc 1620
attctattct ggggggtggg gtggggcagg acagcaaggg ggaggattgg gaagacaata 1680
gcaggcatgc tggggaggat ccgaattcaa cgtacgtagc ttgtaatcat ggtcatagct 1740
gtttcctgtg tgaaattgtt atccgctcac aattccacac aacatacgag ccggaagcat 1800
aaagtgtaaa gcctggggtg cctaatgagt gagctaactc acattaattg cgttgcgctc 1860
actgcccgct ttccagtcgg gaaacctgtc gtgccagctg cattaatgaa tcggccaacg 1920
cgcggggaga ggcggtttgc gtattgggcg ctcttccgct tcctcgctca ctgactcgct 1980
gcgctcggtc gttcggctgc ggcgagcggt atcagctcac tcaaaggcgg taatacggtt 2040
atccacagaa tcaggggata acgcaggaaa gaacatgtga gcaaaaggcc agcaaaaggc 2100
caggaaccgt aaaaaggccg cgttgctggc gtttttccat aggctccgcc cccctgacga 2160
gcatcacaaa aatcgacgct caagtcagag gtggcgaaac ccgacaggac tataaagata 2220
ccaggcgttt ccccctggaa gctccctcgt gcgctctcct gttccgaccc tgccgcttac 2280
cggatacctg tccgcctttc tcccttcggg aagcgtggcg ctttctcata gctcacgctg 2340
taggtatctc agttcggtgt aggtcgttcg ctccaagctg ggctgtgtgc acgaaccccc 2400
cgttcagccc gaccgctgcg ccttatccgg taactatcgt cttgagtcca acccggtaag 2460
acacgactta tcgccactgg cagcagccac tggtaacagg attagcagag cgaggtatgt 2520
aggcggtgct acagagttct tgaagtggtg gcctaactac ggctacacta gaaggacagt 2580
atttggtatc tgcgctctgc tgaagccagt taccttcgga aaaagagttg gtagctcttg 2640
atccggcaaa caaaccaccg ctggtagcgg tggttttttt gtttgcaagc agcagattac 2700
gcgcagaaaa aaaggatctc aagaagatcc tttgatcttt tctacggggt ctgacgctca 2760
gtggaacgaa aactcacgtt aagggatttt ggtcatgaga ttatcaaaaa ggatcttcac 2820
ctagatcctt ttaaattaaa aatgaagttt taaatcaatc taaagtatat atgagtaaac 2880
ttggtctgac agttaccaat gcttaatcag tgaggcacct atctcagcga tctgtctatt 2940
tcgttcatcc atagttgcct gactccccgt cgtgtagata actacgatac gggagggctt 3000
accatctggc cccagtgctg caatgatacc gcgagaccca cgctcaccgg ctccagattt 3060
atcagcaata aaccagccag ccggaagggc cgagcgcaga agtggtcctg caactttatc 3120
cgcctccatc cagtctatta attgttgccg ggaagctaga gtaagtagtt cgccagttaa 3180

tagtttgcgc aacgttgttg ccattgctac aggcatcgtg gtgtcacgct cgtcgtttgg 3240

tatggcttca ttcagctccg gttcccaacg atcaaggcga gttacatgat cccccatgtt 3300

gtgcaaaaaa gcggttagct ccttcggtcc tccgatcgtt gtcagaagta agttggccgc 3360

agtgttatca ctcatggtta tggcagcact gcataattct cttactgtca tgccatccgt 3420

aagatgcttt tctgtgactg gtgagtactc aaccaagtca ttctgagaat agtgtatgcg 3480

gcgaccgagt tgctcttgcc cggcgtcaat acgggataat accgcgccac atagcagaac 3540

tttaaaagtg ctcatcattg gaaaacgttc ttcggggcga aaactctcaa ggatcttacc 3600

gctgttgaga tccagttcga tgtaacccac tcgtgcaccc aactgatctt cagcatcttt 3660

tactttcacc agcgtttctg ggtgagcaaa aacaggaagg caaaatgccg caaaaaaggg 3720

aataagggcg acacggaaat gttgaatact catactcttc ctttttcaat attattgaag 3780

catttatcag ggttattgtc tcatgagcgg atacatattt gaatgtattt agaaaaataa 3840

acaaataggg gttccgcgca catttccccg aaaagtgcca cctgacgtct aagaaaccat 3900

tattatcatg acattaacct ataaaaatag gcgtatcacg aggccctttc gtc 3953

<210> 48

<211> 1706

<212> DNA

<213> Artificial Sequence

<220>

<223> Description of Artificial Sequence: DNA

<400> 48

gcggccgctt caatcgaatt cgttggcatt ccggtactgt tggtaaagcc accatggaag 60

acgccaaaaa cataaagaaa ggcccggcgc cattctatcc gctggaagat ggaaccgctg 120

gagagcaact gcataaggct atgaagagat acgccctggt tcctggaaca attgctttta 180

cagatgcaca tatcgaggtg gacatcactt acgctgagta cttcgaaatg tccgttcggt 240

tggcagaagc tatgaaacga tatgggctga atacaaatca cagaatcgtc gtatgcagtg 300

aaaactctct tcaattcttt atgccggtgt tgggcgcgtt atttatcgga gttgcagttg 360

cgcccgcgaa cgacatttat aatgaacgtg aattgctcaa cagtatgggc atttcgcagc 420
ctaccgtggt gttcgtttcc aaaaaggggt tgcaaaaaat tttgaacgtg caaaaaaagc 480
tcccaatcat ccaaaaaatt attatcatgg attctaaaac ggattaccag ggatttcagt 540
cgatgtacac gttcgtcaca tctcatctac ctcccggttt taatgaatac gattttgtgc 600
cagagtcctt cgatagggac aagacaattg cactgatcat gaactcctct ggatctactg 660
gtctgcctaa aggtgtcgct ctgcctcata gaactgcctg cgtgagattc tcgcatgcca 720
gagatcctat ttttggcaat caaatcattc cggatactgc gattttaagt gttgttccat 780
tccatcacgg ttttggaatg tttactacac tcggatattt gatatgtgga tttcgagtcg 840
tcttaatgta tagatttgaa gaagagctgt ttctgaggag ccttcaggat tacaagattc 900
aaagtgcgct gctggtgcca accctattct ccttcttcgc caaaagcact ctgattgaca 960
aatacgattt atctaattta cacgaaattg cttctggtgg cgctcccctc tctaaggaag 1020
tcggggaagc ggttgccaag aggttccatc tgccaggtat caggcaagga tatgggctca 1080
ctgagactac atcagctatt ctgattacac ccgaggggga tgataaaccg ggcgcggtcg 1140
gtaaagttgt tccatttttt gaagcgaagg ttgtggatct ggataccggg aaaacgctgg 1200
gcgttaatca aagaggcgaa ctgtgtgtga gaggtcctat gattatgtcc ggttatgtaa 1260
acaatccgga agcgaccaac gccttgattg acaaggatgg atggctacat tctggagaca 1320
tagcttactg ggacgaagac gaacacttct tcatcgttga ccgcctgaag tctctgatta 1380
agtacaaagg ctatcaggtg gctcccgctg aattggaatc catcttgctc caacacccca 1440
acatcttcga cgcaggtgtc gcaggtcttc ccgacgatga cgccggtgaa cttcccgccg 1500
ccgttgttgt tttggagcac ggaaagacga tgacggaaaa agagatcgtg gattacgtcg 1560
ccagtcaagt aacaaccgcg aaaaagttgc gcggaggagt tgtgtttgtg gacgaagtac 1620
cgaaaggtct taccggaaaa ctcgacgcaa gaaaaatcag agagatcctc ataaaggcca 1680
agaagggcgg aaagatcgcc gtgtaa 1706

<210> 49
<211> 1115
<212> DNA

<213> Artificial Sequence

<220>

<223> Description of Artificial Sequence: CMV5 promoter

<400> 49

gaccgccatg ttgacattga ttattgacta gttattaata gtaatcaatt acggggtcat 60

tagttcatag cccatatatg gagttccgcg ttacataact tacggtaaat ggcccgcctg 120

gctgaccgcc caacgacccc cgcccattga cgtcaataat gacgtatgtt cccatagtaa 180

cgccaatagg gactttccat tgacgtcaat gggtggagta tttacggtaa actgcccact 240

tggcagtaca tcaagtgtat catatgccaa gtccgccccc tattgacgtc aatgacggta 300

aatggcccgc ctggcattat gcccagtaca tgaccttacg ggactttcct acttggcagt 360

acatctacgt attagtcatc gctattacca tggtgatgcg gttttggcag tacaccaatg 420

ggcgtggata gcggtttgac tcacggggat ttccaagtct ccaccccatt gacgtcaatg 480

ggagtttgtt ttggcaccaa aatcaacggg actttccaaa atgtcgtaat aaccccgccc 540

cgttgacgca aatgggcggt aggcgtgtac ggtgggaggt ctatataagc agagctcgtt 600

tagtgaaccg tcagatcctc actctcttcc gcatcgctgt ctgcgagggc cagctgttgg 660

gctcgcggtt gaggacaaac tcttcgcggt ctttccagta ctcttggatc ggaaacccgt 720

cggcctccga acgtactccg ccaccgaggg acctgagcca gtccgcatcg accggatcgg 780

aaaacctctc gagaaaggcg tctaaccagt cacagtcgca aggtaggctg agcaccgtgg 840

cgggcggcag cgggtggcgg tcggggttgt ttctggcgga ggtgctgctg atgatgtaat 900

taaagtaggc ggtcttgagc cggcggatgg tcgagagatc cagctgttgg ggtgagtact 9600

ccctctcaaa agcgggcatg acttctgcgc taagattgtc agtttccaaa aacgaggagg 1020

atttgatatt cacctggccc gatctggcca tacacttgag tgacaatgac atccactttg 1080

cctttctctc cacaggtgtc cactcccagg tccaa 1115

<210> 50

<211> 795

<212> DNA

<213> Artificial Sequence

<220>

<223> Description of Artificial Sequence: neomycine phosphotransferase gene

<400> 50

atgattgaac aagatggttt acatgcaggt tcaccagcag catgggtaga acgattattt 60

ggttatgatt gggcacaaca aacaatcggt tgttcagatg cagcagtatt tcgattatca 120

gcacaaggtc gaccagtatt atttgtaaaa acagatttat caggtgcatt aaatgaatta 180

caagatgaag cagcacgatt atcatggtta gcaactactg gtgtaccatg tgcagcagta 240

ttagatgtag taactgaagc aggtcgagat tggttattat taggtgaagt accaggtcaa 300

gatttattat catcacattt agcaccagca gaaaaagtat caatcatggc agatgcaatg 360

cgacgattac atacattaga tccagcaaca tgtccatttg atcatcaagc aaaacatcga 420

atcgaacgag cacgaactcg aatggaagca ggtttagtag atcaagatga tttagatgaa 480

gaacatcaag gtttagcacc agcagaatta tttgcacgat taaaagcacg aatgccagat 540

ggtgaagatt tagtagtaac tcatggtgat gcatgtttac caaatatcat ggtagaaaat 600

ggtcgatttt caggttttat cgattgtggt cgattaggtg tagcagatcg atatcaagat 660

atagcattag caactcgaga tattgcagaa gaattaggtg gtgaatgggc agatcgattt 720

ttagtattat atggtatcgc agcaccagat tcacaacgaa tcgcatttta tcgattatta 780

gatgaatttt tttga 795

<210> 51

<211> 564

<212> DNA

<213> Artificial Sequence

<220>

<223> Description of Artificial Sequence: dihydrofolate reductase gene

<400> 51

atggtacgac cattaaattg tatcgtagca gtatcacaaa atatgggtat tggtaaaaat 60

ggtgatttac catggccacc attacgaaat gaatttaaat attttcaacg aatgactaca 120

acttcatcag tagaaggtaa acaaaattta gtaattatgg gtcgaaaaac atggttttca 180

attccagaaa aaaatcgacc attaaaagat cgaattaata tagtattatc acgagaatta 240

aaagaaccac cacgaggtgc acatttttta gcaaaatcat tagatgatgc attacgatta 300

attgaacaac cagaattagc atcaaaagta gatatggtat ggatagtagg tggttcatca 360

gtatatcaag aagcaatgaa tcaaccaggt catttacgat tatttgtaac acgaatcatg 420

caagaatttg aatcagatac tttttttcca gaaattgatt taggtaaata taaattatta 480

ccagaatatc caggtgtatt atcagaagta caagaagaaa aaggtathaa gtataaattt 540

gaagtatatg aaaaaaaaga ttaa 564

## Description

### TECHNICAL FIELD

The present invention relates to a vector DNA for animal cells, which realizes high-level production of recombinant proteins using an animal cell as a host. The vector of the present invention is particularly useful for expressing a protein which cannot be produced as an active protein when a bacterium, eukaryotic microorganism or insect cell is used as a host.

### BACKGROUND ART

A large number of vectors used for production of recombinant proteins have been developed, and such vectors enable the production of such recombinant proteins at high yield in an expression system in which a bacterium, eukaryotic microorganism or insect cell is used as a host. However, in the expression system in which these hosts are used, there may be problems regarding modification of proteins, or the characteristics of mammalian proteins may be lost. Thus, for example, in the case of a protein having a biological activity which depends on sugar chains, it is necessary to produce the protein using a mammalian cell as a host. Although there is such a requirement, the productivity of recombinant proteins is generally found to be low in an expression system in which a mammalian cell is used as a host. Also, there is a problem regarding the stability of an introduced gene in many cases.

Examples of a case of producing a recombinant protein using a mammalian cell as a host include erythropoietin (Japanese Patent Laid-Open No. 2002-529100) and IFN-β (Japanese Patent Laid-Open No. 7-265084). In addition, there have been many reports regarding production of a genetically recombinant monoclonal antibody (Japanese Patent Laid-Open Nos. 7-67648, 6-30788 and 6-217786).

### DISCLOSURE OF THE INVENTION

Improvement of productivity of a recombinant protein using a mammalian cell as a host is not only very important for the production of therapeutic agents or diagnostic agents, but it largely contributes to research for development of these agents. Thus, the development of a vector that enables high-level productive strains to be easily obtained, using, as a host, a mammalian cell, and especially, a Chinese hamster ovary cell (CHO cell), is considered to be an urgent necessity.

In order to easily obtain high-level productive strains, it is adequate to allow high-level productive strains producing a large number of substances of interest to exist in a small number of transformed cells. Thus, it becomes easy to select high-level productive clones.

It has been found that the expression level of a gene differs significantly depending on positions on a chromosome (Annu. Rev. Cell Biol., 6, p. 679, 1990). In order to obtain high-level productive strains, it seems to be important that a vector DNA used in transformation is targeted to a site on a chromosome which allows high gene expression level, considering the properties or performances of the vector DNA.

As stated above, an object of the present invention is to provide an expression vector which enables easy obtainment of high-level productive strains, using a mammalian cell, and especially, a Chinese hamster ovary cell (CHO cell) as a host.

The present inventors have developed a vector having a mechanism in which strains wherein a gene cassette of interest is incorporated into a position which allows a high gene expression level on the chromosome of a host cell eventually survive as neomycine-resistant strains. In order to enable targeting to a position which allows a high gene expression level on a chromosome, it is important to set the expression level of a neomycine phosphotransferase (hereinafter referred to as NPT) gene to be infinitesimal. Moreover, when the above vector has a dihydrofolate reductase (hereinafter referred to as DHFR) gene and is designed in such a way that the expression level of the gene is controlled, it enables efficient gene amplification by stimulation of methotrexate. In the present invention, a vector meeting the two above requirements has been developed. As a result, a vector that enables high-level and stable production of proteins can be produced, thereby completing the present invention.

Thus, the present invention provides the followings:
(1) An expression vector inducing high productivity of genetically recombinant proteins in animal host cells, which comprises a highly expression-inducible promoter, a multicloning site for insertion of gene and a polyadenylation signal sequence in this order from upstream, and further comprises downstream thereof a drug resistant gene that does not have a promoter capable of acting in animal cells.
(2) The expression vector according to (1) above, wherein the highly expression-inducible promoter is a human cytomegalovirus Major Immediately-Early antigen promoter, CMV5 promoter (a synthetic chimeric promoter), a β-actin promoter, or an SV40 early promoter.
(3) The expression vector according to (1) or (2) above, wherein the drug resistant gene is a neomycine resistant gene, codon-disoptimized neomycine resistant gene, a hygromycin resistant gene, a zeocin resistant gene, or a blasticidin resistant gene.
(4) The expression vector according to any one of (1) to (3) above, wherein the drug resistant gene is a neomycine phosphotransferase gene.
(5) The expression vector according to any one of (1) to (4) above, wherein the drug resistant gene is a neomycine phosphotransferase gene derived from *Escherichia coli* transposon Tn5.
(6) The expression vector according to any one of (1) to (5) above, wherein a trace amount of the messenger RNA or protein of a drug resistant gene is generated by a read-through effect which is achieved by the fact that the drug resistant gene exists downstream of the highly expression-inducible promoter blocked by the polyadenylation signal sequence.
(7) The expression vector according to any one of (1) to (6) above, which further comprises, downstream of the drug resistant gene, a dihydrofolate reductase gene or codon-disoptimized dihydrofolate reductase gene that does not have a promoter capable of acting in animal cells.
(8) The expression vector according to any one of (1) to (7) above, which further comprises, downstream of the drug resistant gene, a highly expression-inducible promoter, a multicloning site for insertion of gene and a polyadenylation signal sequence in this order from upstream, and further comprises downstream thereof a dihydrofolate reductase gene or codon-disoptimized dihydrofolate reductase gene that does not have a promoter capable of acting in animal cells.
(9) The expression vector according to any one of (1) to (6) above, which comprises a dihydrofolate reductase gene or codon-disoptimized dihydrofolate reductase gene ligated downstream of a promoter with low expression inductivity.
(10) The expression vector according to any one of (7) to (9) above, wherein the promoter with low expression inductivity is a promoter derived from a gene of a protein that is generally expressed in trace amounts in mammalian cells, a promoter derived from a viral antigen gene that hardly ever infects mammals, and a promoter obtained by removing an enhancer sequence from the above promoters.
(11) A recombinant expression vector which is obtained by incorporating a gene of interest into the multicloning site for insertion of gene of the expression vector according to any one of (1) to (6) above.
(12) A recombinant expression vector which is obtained by incorporating a gene of interest into the multicloning site for insertion of gene of the expression vector according to any one of (7) to (10) above.
(13) A transformant comprising the expression vector according to any one of (1) to (10) above or the recombinant expression vector according to (11) or (12) above.
(14) A method for producing a transformant that highly expresses a gene of interest, which comprises introducing the recombinant expression vector according to (11) or (12) above into animal host cells.
(15) A method for obtaining a transformant capable of stably producing proteins in a serum free medium, which comprises introducing the recombinant expression vector according to (11) or (12) above into animal host cells so as to obtain a transformant, and acclimating the obtained transformant to the serum free medium.
(16) A method for amplifying a gene of interest, which comprises introducing the recombinant expression vector according to (12) above into a dihydrofolate reductase-deficient CHO cell, and culturing the cell in the presence of methotrexate.

### BRIEF DESCRIPTION OF THE DRAWINGS

Figure 1 is a flow chart for construction of a neomycine resistant vector pSV/GKT-Neo.
Figure 2 is a flow chart for construction of a neomycine resistant/methotrexate resistant vector pND.
Figure 3 is a flow chart for construction of a cassette plasmid pCB used for expression of a desired gene.
Figure 4 is a flow chart for construction of a foreign gene expression vector pNOS-GKT2 having an NPT gene cistron that does not have a transcription promoter acting in mammalian cells.
Figure 5 is a flow chart for construction of a foreign gene expression vector pNOS-GKT2B, in which bGH-poly A for termination of transcription is located upstream of the NPT gene cistron having no promoters.
Figure 6 is a flow chart for construction of a foreign gene expression vector pNOS-GKT2B comprising a rabbit β globin gene intron for improvement of translation efficiency.
Figure 7 is a map of the expression vector pNOS-GKT2B of the present invention.
Figure 8 is a flow chart for construction of an hG-CSF expression vector pNOS-GKT2B-R/hG-CSF.
Figure 9 is an example of detection of hG-CSF by the dot blot method, which was produced by clones transfected with the expression vector pNOS-GKT2B of the present invention.
Figure 10 is a distribution of the expression level of hG-CSF of each clone transfected with the expression vector pNOS-GKT2B of the present invention.
Figure 11 is a map of the expression vector pNOS of the present invention used in Example 4.
Figure 12 is an example of detection of hG-CSF by the dot blot method, which was produced by clones transfected with the expression vector pNOS/human G-CSF of the present invention.
Figure 13 is another example of detection of hG-CSF by the dot blot method, which was produced by clones tranfected with the expression vector pNOS/human G-CSF of the present invention.
Figure 14 is a distribution of the expression level of hG-CSF of each clone transfected with the expression vector pNOS/human G-CSF of the present invention.
Figure 15 is a flow chart for preparation of CMV5 promoter.
Figure 16 is a flow chart for preparation of CMV5 promoter.
Figure 17 is a flow chart for preparation of pCMV5-luciferase.
Figure 18 is the comparison of the activity of the CMV5 promoter with CMV promoter by luciferase assay.

### BEST MODE FOR CARRYING OUT THE INVENTION

The embodiments of the present invention will be described in detail below.

If an introduced drug resistant gene (e.g., an NPT gene) is not expressed or its expression level is too low, a host cell into which vector DNA has been introduced cannot obtain resistance to the corresponding drug (e.g., neomycine (hereinafter referred to as NEO)). However, in studies that the present inventors have been made to date, in all cases of using vector DNA comprising a common NPT gene, including commercially available vectors, numerous NEO resistant strains have been obtained. From these results, it has been assumed that the expression of an NPT gene provided from vector DNA contributes as a selective marker for determining whether or not a cell obtained as a result of transfection is a transformant, but that it has no effects other than that.

The present inventors have limited the induction of NPT expression to an extreme extent, so that they have designed NPT such that NPT is not expressed at a level necessary for obtaining resistance to NEO, as long as an NPT gene cistron is not incorporated into a site which allows a high gene expression level on the chromosome of a host cell. Thus, they have designed vector DNA such that a transformed host cell does not acquire resistance to NEO and cannot survive in a medium containing a certain amount of NEO, as long as the vector DNA is introduced into a site which allows an extremely high level of gene expression on the chromosome. There have been several reports on the use of a weak promoter for NPT gene cistrons of vector DNA (Mol. Cell Biol., 6, p. 2593, 1986; Mol. Cell Biol., 7, p. 1296, 1987; DNA, 7, p. 651, 1988). However, it is difficult to think that highly productive transformants are effectively and sufficiently selected in these cases.

The present invention is based on the principle that a DNA fragment comprising an NPT gene cistron derived from *Escherichia coli* transposon Tn5, while it has no promoters having transcriptional activity in mammalian cells, is allowed to locate downstream of a promoter having transcriptional activity in mammalian cells and the following optimal polyadenylation signal haing strong transcription termination activity, so that the NPT expression mechanism in the transformed host cells is significantly damaged and attenuated. Since the NPT gene cistron of the present invention has no promoters, it is defined as a "promoterless NPT gene cistron."

Thus, the present invention provides an expression vector, which comprises a highly expression-inducible promoter, a multicloning site for insertion of gene and a polyadenylation signal sequence in this order from upstream, and further comprises downstream thereof a drug resistant gene that does not have a promoter capable of acting in animal cells. The expression vector of the present invention can induce high productivity of genetically recombinant proteins in animal host cells.

In the present invention, examples of a drug resistant gene may include a neomycine resistant gene, a hygromycin resistant gene, a zeocin resistant gene, and a blasticidin resistant gene. Particularly preferably, the drug resistant gene is a neomycine phosphotransferase gene. Most preferably, it is a neomycine phosphotransferase gene derived from *Escherichia coli* transposon Tn5. In the present invention, a neomycine phosphotransferase gene of SEQ ID NO.50 having a weak expression level, which is prepared by substituting a part or all of the codons of neomycine phosphotransferase gene with codons which are used at a low frequency in CHO cells, defined as codon-disoptimized neomycine resistant gene, can be used as a drug resistant marker to construct a foreign gene expression vector.

In the present invention, a trace amount of the messenger RNA or protein of drug resistant gene is generated by a read-through effect obtained by the fact that the drug resistant gene exists downstream of the highly expression-inducible promoter blocked by the polyadenylation signal sequence.

Based on properties of a drug resistant gene cistron, the incorporation of the expression vector of the present invention into a high expression site on a host cell chromosome is achieved by selection of survival cells in a medium containing the corresponding drug. In this case, it is necessary to strongly induce the expression of a protein of interest at the above site on the chromosome. Accordingly, a promoter comprised in a multicloning site (hereinafter referred to as MCS) for insertion of a protein gene and a polyadenylation signal (hereinafter referred to as polyA) are selected from those having the strongest expression inductivity. Examples of such promoters may include a human cytomegalovirus Major Immediately-Early antigen promoter (hCMV MIE: Cell, 41, p. 521, 1985), a β-actin promoter (Proc. Natl. Acad. Sci. USA, 84, p. 4831, 1987), and an SV40 early promoter. In the present invention, CMV 5 promoter having a nucleotide sequence of SEQ ID NO.49 which is prepated by the method according to Massie B et al. (Journal of Virology, 1998 Mar;72(3):2289-96. Inducible overexpression of a toxic protein by an adenovirus vector with a tetracycline-regulatable expression cassette. Massie B, Couture F, Lamoureux L, Mosser DD, Guilbault C, Jolicoeur P, Belanger F, Langelier Y.) can be used instead of the aforementioned CMV promoter to construct a foreign gene expression vector.

An example of such poly A signals may include a poly A sequence derived from human growth hormone. In the present specification, a cistron having MCS into which the above gene of protein of interest is incorporated is referred to as an "expression cassette".

The present invention also relates to a transformant obtained by introducing into a host the expression vector of the present invention into which a gene of interest is incorporated, and a method for producing the transformant.

Moreover, the expression vector of the present invention preferably has a DHFR gene cistron. For this expression vector, a dihydrofolate reductase-deficient CHO cell can be used as a host cell. A gene in the vector DNA gene is introduced into the above host cell, and thereafter, the cell is cultured in the presence of methotrexate, so that the gene contained in the above vector DNA can be efficiently amplified. In the present invention, a dihydrofolate reductase gene of SEQ ID NO.51 having a weak expression level, which is prepared by substituting a part or all of the codons of dihydrofolate reductase gene with codons which are used at a low frequency in CHO cells, defined as codon-disoptimized dihydrofolate reductase gene, can be used as a selective marker to construct a foreign gene expression vector. The dihydrofolate reductase gene of SEQ ID NO.51 having a weak expression level can be used in combination with the neomycine phosphotransferase gene of SEQ ID NO.50 having a weak expression level.

With regard to a DHFR gene cistron, as in the case of the NPT gene cistron, the DHFR gene cistron, while it has no promoters capable of expressing in mammalian cells, is allowed to locate downstream of multicloning site for insertion of genes having a highly expression-inducible promoter and the following optimal polyadenylation signal sequence, and it is used. Alternatively, a promoter whose expression inductivity is reduced, for example, a promoter obtained by removing an enhancer region from an SV40 viral antigen promoter generally noninfectious to CHO cells, is ligated to the DHFR gene cistron, and it is then used. The present inventors have considered that, in order to promote gene amplification, it is more effective to reduce the expression inductivity of a DHFR gene or to repress such inductivity, so as to make MTX contained in a medium promote the amplification of vector DNA incorporated into a host cell chromosome.

Moreover, the present invention provides a method for obtaining a high-level productive cell clone capable of producing a high level of proteins, which comprises transforming a host cell by gene transfer using the above vector DNA having the DHFR gene cistron into which a protein gene is incorporated, and amplifying the vector DNA incorporated into the host cell chromosome.

Furthermore, the present invention provides a method for obtaining a high-level productive cell clone capable of stably producing a high level of proteins in a serum free medium, which comprises transforming a host cell by gene transfer using the above vector DNA into which a protein gene is incorporated, and acclimating the obtained transformant to a serum free medium.

Still further, the present invention provides vector DNA, which has the above-described DHFR gene cistron and a multicloning site for insertion of gene located between a highly expression-inducible promoter and an optimal polyadenylation signal. This vector DNA can be used to construct vector DNA comprising the above-described promoterless NPT gene cistron.

In the present invention, the term "cistron" is used to mean a unit, which expresses proteins by transcription and translation, using a promoter, a structural gene and a polyadenylation signal (poly A), as a basic structure. The cistron may also comprise, as an insertion sequence, a DNA sequence associated with any of them or any given DNA sequence. The term "highly expression-inducible promoter" is used to mean a promoter commonly used for protein expression in ordinary vector DNA, and it preferably means a promoter having particularly high expression inductivity, such as a human cytomegalovirus Major Immediately-Early antigen promoter, β-actin promoter, or SV40 early promoter.

The vector DNA of the present invention can be obtained by incorporating an NPT gene cistron and an expression cassette into a backbone vector, and by further incorporating a DHFR gene cistron, if desired. The backbone vector is not particularly limited. For example, vector DNA (pUC series, Gene, 33: p. 103, 1985, etc.) having an *Escherichia coli* replication origin (ColE1 ori) can be used as such a backbone vector.

An example of a method of using the vector of the present invention may include a method comprising incorporating a gene of protein into the MCS of the vector of the present invention, introducing the gene into a host cell by the cationic liposome method, selecting strains surviving in a medium containing NEO, so as to obtain cell clones capable of producing a high level of proteins. A majority of cells obtained by selecting strains surviving in a medium containing NEO has already achieved a relatively high expression level. However, in order to select cells having further high productivity from among them, the expression level of proteins may also be measured. The obtained cells having high productivity can be repeatedly subjected to limiting dilution, followed by subculture and stabilization. When further higher productive strains are required to be obtained, a vector further comprising a DHFR gene cistron is used as the vector of the present invention. MTX is added to a medium, and the DHFR gene incorporated into a host cell is stimulated by MTX, so as to amplify the gene.

Moreover, a more specific method of using the expression vector (pNOS-GKT2B or pNOS-GKT2B-R) of the present invention that will be described later in examples comprises the following steps:
(1) A gene of a substance (protein) of interest is cloned, and then (2) it is incorporated into the multicloning site (MCS) of the expression vector (pNOS-GKT2B or pNOS-GKT2B-R) of the present invention to make expression construct. (3) The expression construct is amplified in *Escherichia coli*, (4) the gene is introduced in a CHO cell (DHRF negative) by the lipofectin method or electroporation, and (5) G418 is added to a medium followed by culture, so as to select G418 resistant cells. (6) The obtained cells are repeatedly cloned while culturing them, (7) clones producing a high amount of substances of interest are selected, and (8) the culture is continued until the growth rate becomes faster and is stabilized. (9) FCS is gradually removed from the FCS-added medium containing stabilized clones, so as to acclimate the clones to a serum free medium. (10) The DNA of introduced construct is amplified by stepwise addition of MTX (15 nM-1 µM) to the medium containing the highly productive clones, and then (11) highly productive clones are selected again, and (12) the culture is continued until the growth rate becomes faster and is stabilized. (13) FCS is gradually removed from the FCS-added medium containing stabilized clones, so as to acclimate the clones to a serum free medium. It is to be noted that the conversion of the medium into a serum free medium may be carried out either in the step (9) or (13).

The method for producing the expression vector of the present invention and the method of using the same will be further described in the following examples. A person skilled in the art can readily substitute the constituent elements used in the present examples, such as a starting plasmid or promoter, with items equivalent thereto. Accordingly, the examples are not intended to limit the scope of the present invention.

### EXAMPLES

### Example 1: Construction of the expression vector of the present invention

### (1) Production of neomycine resistant vector [pSV/GKT-Neo]

An NEO vector [pSV/GKT-Neo] comprises an *Escherichia coli* replication origin (ColE1 ori) derived from a plasmid pUC18 (Gene, 33, p. 103, 1985), and a neomycine phosphotransferase (NPT) gene cistron. The *Escherichia coli* replication origin was amplified from the plasmid pUC 18 by the PCR method, using primers having nucleotide sequences shown in SEQ ID NOS: 1 and 2. The PCR was carried out under the following conditions. A kit commercially available from Takara Shuzo Co., Ltd. was used as a PCR kit, and as a 10 x reaction buffer and a dNTP solution, items attached to the kit were used. First, the 10 x reaction buffer, the dNTP solution, 1 µl each of 100 µM primers shown in SEQ ID NOS: 1 and 2, 1 µl of template DNA and 0.5 µl of Pyrobest DNA polymerase (5 U/µl) were added to 36.5 µl of sterile distilled water, and the obtained mixture was completely blended. Thereafter, a PCR reaction was carried out using Takara Thermal Cycler PERSONAL. This is to say, after heating at 94°C for 5 minutes, a cycle consisting of the following 3 steps, denaturation (94°C, 30 seconds), primer annealing (55°C for 30 seconds), and elongation (72°C, 1 minute), was repeated 35 times, and finally the reaction product was heated at 72°C for 5 minutes, so as to terminate the reaction. It is to be noted that the PCR reactions described hereinafter were carried out using the same reaction cycle as described above. After completion of the reaction, the obtained PCR product consisting of approximately 600 base pairs was cleaved with restriction enzymes (all the restriction enzymes described below were manufactured by NEB), that is, 1 µl of *Sac*II (20000 U/µl) and 1 µl of *Cla*I (5000 U/µl), at 37°C for 2.5 hours. A total amount of the reaction solution obtained by the treatment with the restriction enzymes was added to 1% agarose gel, followed by electrophoresis at 60 mA for 25 minutes. A DNA band consisting of approximately 600 base pairs was recovered together with the gel, and it was frozen at -135°C for 10 minutes. Thereafter, it was subjected to centrifugal separation, and a DNA fragment was recovered from the supernatant (ColE1 ori cassette; 36 ng/µl). The following restriction enzyme treatment and recovery of DNA fragments were carried out in the same manner as described above.

Next, 3 µg of a plasmid pSV(D)S/Neo (Japanese Patent Laid-Open No. 10-179169) was cleaved with 1 µl each of restriction enzymes *Sac*II (20000 U/µl) and *Cla*I (5000 U/µl), and a DNA fragment (NEO cassette 1; 28 ng/µl) consisting of approximately 2200 base pairs was prepared, which had an NPT gene cistron downstream of a monkey virus 40 (SV40) early promoter from which an enhancer portion had been removed. The two obtained fragments were ligated to each other using a Takara DNA ligation kit ver. 2 (manufactured by Takara Shuzo Co., Ltd.), so as to prepare pSVNEO/Ori'. Practically, 1 µl of a ColE1 ori cassette was mixed with 1 µl of an NEO cassette 1, and a Takara DNA ligation kit ver. 2-solution I was then added to the mixture in an amount equal to the mixed solution. The obtained mixture was reacted at 16°C for 30 minutes, so as to ligate these DNA. Subsequently, the DNA was transformed in *Escherichia coli* (*E. Coli*) XL-1BlueTM competent cells according to the rubidium-chloride method. The obtained *Escherichia coli* colonies on the LB-agar (Difco) containing 50 µg/ml kanamycin sulfate (Sigma) were inoculated into a Terrific broth (containing per liter: 12 g of bacto-tryptone (manufactured by Difco), 24 g of bacto yeast extract (manufactured by Difco), 4 ml of glycerol (manufactured by Wako Pure Chemical Industries, Ltd.), 2.31 g of KH₂PO₄ (manufactured by Wako Pure Chemical Industries, Ltd.), and 12.5 g of K₂HPO₄ (manufactured by Wako Pure Chemical Industries, Ltd.)), which contained kanamycin sulfate (50 µg/ml; Sigma). After culture for about 20 hours, cells were collected, and plasmids were isolated by the alkali-SDS method (Current Protocols in Molecular Biology, p.1-6-3, 1986), so as to prepare pSVNEO/Ori'. It is to be noted that all of the ligation, transformation and the isolation of plasmids described hereinafter were carried out by the same methods as described above.

Next, pSVNEO/Ori' was cleaved with 1 µl each of restriction enzymes *Apa*I (1000 U/µl) and *Cla*I (5000 U/µl), and then purified. To the obtained DNA fragment (38 ng/µl) consisting of approximately 2800 base pairs, a linker (pSVNEO linker) having multiple restriction enzyme cleavage sites obtained by annealing newly synthesized oligonucleotides having nucleotide sequences shown in SEQ ID NOS: 3 and 4 was ligated. It was then transformed, and plasmids were isolated, so as to prepare pSVNEO/Ori. The linker was produced by placing 50 µl each of oligonucleotides (100 µM) shown in SEQ ID NOS: 3 and 4 into a microtube (1.5 ml; manufactured by Tref), and heating it with heat block at 70°C for 5 minutes, followed by cooling it to 25°C for about 1 hour. Hereinafter, a linker was produced in the same manner as described above.

At the same time, a DNA fragment comprising a promoter sequence controlling the expression inductivity of the NPT gene cistron in *Escherichia coli* was amplified from a plasmid pSV2NEO (manufactured by Stratagene) by PCR using primers having nucleotide sequences shown in SEQ ID NOS: 5 and 6. A kit commercially available from Takara Shuzo Co., Ltd. was used as a PCR kit, and the attached items were used as 10 x reaction buffer, a dNTP solution and 10 x magnesium chloride solution. First, 5 µl of 10 x reaction buffer, 5 µl of the dNTP solution, 5 µl of 10 x magnesium chloride solution, 1 µl each of 100 µM primers shown in SEQ ID NOS: 5 and 6, 1 µl of template DNA and 0.5 µl of LA Taq DNA polymerase (5 U/µl) were added to 31.5 µl of sterile distilled water, and the obtained mixture was completely blended. Thereafter, a PCR reaction was carried out using Takara Thermal Cycler PERSONAL. It is to be noted that PCR reactions using LA Taq DNA polymerase described hereinafter were carried out applying the same mixing ratio. The obtained PCR product consisting of approximately 400 base pairs was run on agarose gel, and then separated and purified (GKT fragment; 58 ng/µl). It was then ligated with a plasmid for cloning PCR products pT7Blue(R) (50 ng/µl; Novagen) by ligation, followed by transformation and the isolation of plasmids, so as to prepare pT7B-GKT.

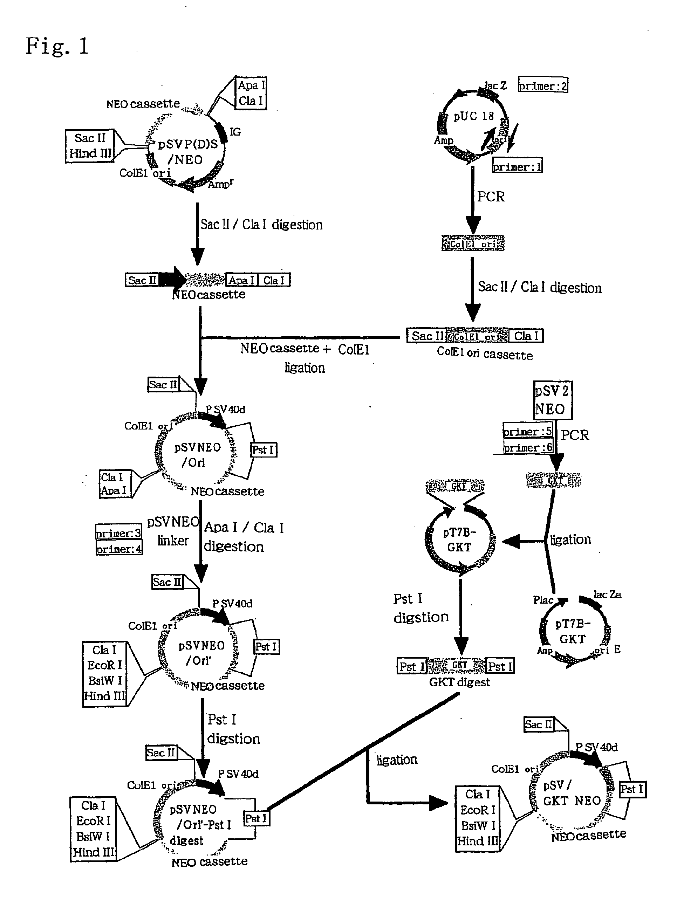

Subsequently, 6 µg of pSVNEO/Ori was cleaved with 1 µl of restriction enzyme *Pst*I (20000 U/µl). The obtained fragment (38 ng/µl) consisting of approximately 2800 base pairs and comprising an *Escherichia coli* replication origin, an SV40 early promoter from which an enhancer portion had been removed, and a partial sequence of the NPT gene cistron, was ligated to a fragment (GKT cassette; 9 ng/µl) consisting of approximately 400 base pairs and comprising a partial sequence of the NPT gene cistron, which was obtained by cleaving 6 µg of pT7B-GKT with *Pst*I. Thereafter, the obtained product was transformed, and plasmids were isolated, so that pSV/GKT-Neo was produced. A flow chart for construction of [pSV/GKT-Neo] is shown in Figure 1.

### (2) Production of neomycine resistant/methotrexate resistant vector [pND]

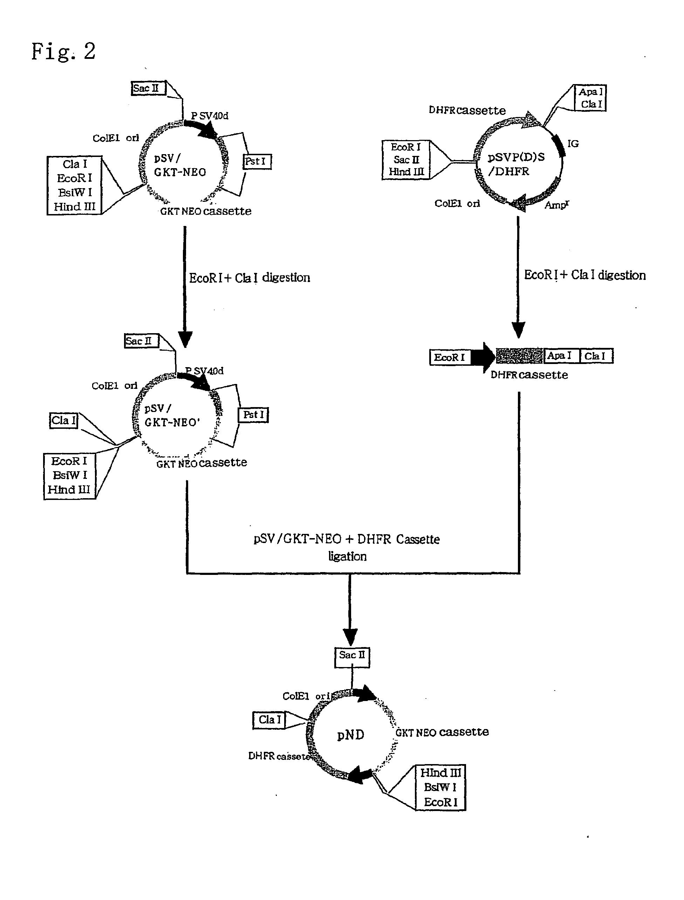

5 µg of the above pSV/GKT-Neo was cleaved with 1 µl each of restriction enzymes EcoRI (20000 U/µl) and *Cla*I (5000 U/µl). The obtained fragment was run on agarose gel, and it was then separated and purified, so as to obtain a fragment pSV/GKT-Neo cassette (44 ng/µl) consisting of approximately 3000 base pairs. Moreover, pSVP(D)S/DHFR (Japanese Patent Laid-Open No. 10-179169) was cleaved with 1 µl each of restriction enzymes EcoRI (20000 U/µl) and *Cla*I (5000 U/µl), so as to obtain a fragment (DHFR cassette; 25 ng/µl) consisting of approximately 1800 base pairs, and comprising an SV40 early promoter from which an enhancer portion had been removed and a dihydrofolate reductase (DHFR) gene cistron. The pSV/GKT-Neo cassette was ligated with the DHFR cassette by ligation, followed by transformation and the isolation of plasmids, so as to obtain pND. A flow chart for construction of [pND] is shown in Figure 2.

### (3) Production of cassette plasmid [pCB] for desired gene expression

This vector was comprised of a promoter, a multicloning site (MCS) for insertion of gene of protein of interest, and a polyadenylation signal. A human cytomegalovirus (hCMV) MIE antigen promoter (PCMV) was used herein as a promoter, which is considered as a promoter having the highest expression inductivity. In addition, a bovine growth hormone poly A signal (bGH-poly A) was used as a poly A signal, which has been reported to be highly productive and excellent in reproducibility.

First, DNA was amplified from a plasmid pRc/CMV (manufactured by Invitrogen) by PCR with LA Taq DNA polymerase, using primers having nucleotide sequences shown in SEQ ID NOS: 7 and 8. The thus amplified DNA was run on agarose gel, and it was then separated and purified, so as to obtain a PCMV fragment consisting of approximately 650 base pairs. The PCMV fragment was ligated to a plasmid pT7Blue(R), followed by transformation and the isolation of plasmids, so as to produce pT7B-PCMV. At the same time, DNA was amplified from the plasmid pRc/CMV by PCR with LA Taq DNA polymerase, using primers having nucleotide sequences shown in SEQ ID NOS: 9 and 10, so as to obtain a bGH-poly A fragment consisting of approximately 650 base pairs. The thus obtained fragment was run on agarose gel, and it was then separated and purified. Thereafter, the fragment was ligated to a plasmid pT7Blue(R), followed by transformation and the isolation of plasmids, so as to produce pT7B-bGH-poly A. At the same time, 5 µg of a plasmid pUC 18 was cleaved with 1 µl each of restriction enzymes *Eco*RI (20000 U/µl) and *Hind*III (20000 U/µl). To the obtained DNA fragment, a linker (CB linker) having multiple restriction enzyme-cleavage sites obtained by annealing newly synthesized oligonucleotides having nucleotide sequences shown in SEQ ID NOS: 11 and 12 was ligated, so as to prepare pUC18-CB-linker.

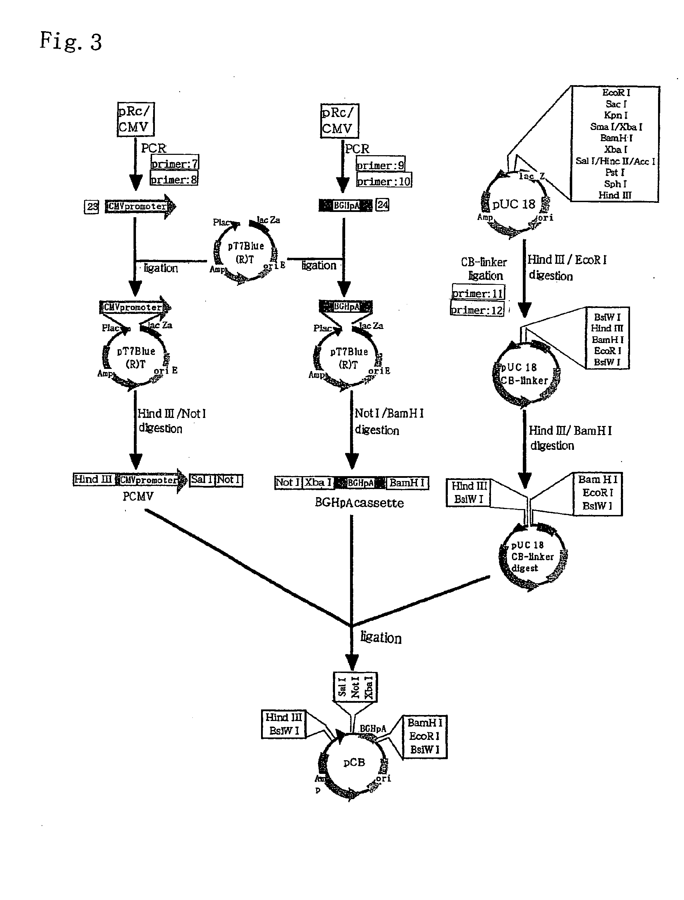

Thereafter, 4.5 µg of the pUC18-CB-linker was cleaved with 1 µl each of restriction enzymes *Bam*HI (20000 U/µl) and *Hind*III (20000 U/µl) to obtain a DNA fragment consisting of approximately 2700 base pairs. The DNA fragment was run on agarose gel, and it was then separated and purified, so as to obtain a pUC18-CB-linker cassette (26 ng/µl). Moreover, 4.7 µg of the pT7B-PCMV was cleaved with 1 µl each of restriction enzymes *Not*I (10000 U/µl) and *Hind*III (20000 U/µl), and the obtained fragment was run on agarose gel, and it was then separated and purified, so as to obtain a DNA fragment (PCMV cassette; 12 ng/µl) consisting of approximately 650 base pairs and comprising PCMV. Furthermore, 1.3 µg of the pT7B-bGH-poly A was cleaved with 1 µl each of restriction enzymes *Not*I (10000 U/µl) and *Bam*HI (20000 U/µl), and the obtained fragment was run on agarose gel, and it was then separated and purified, so as to obtain a DNA fragment (bGH-poly A cassette; 2 ng/µl) consisting of approximately 650 base pairs and comprising bGH-poly A. The pUC18-CB-linker cassette, the PCMV cassette and the bGH-poly A cassette were ligated with one another by ligation, followed by transformation and the isolation of plasmids, so as to achieve [pCB]. A flow chart for construction of a cassette plasmid [pCB] for the expression of a desired gene is shown in Figure 3.

### (4) Production of foreign gene expression vector [pNOS-GKT2] having NPT gene cistron having no transcription promoters acting in mammalian cells

5 µg of the pCB was cleaved with 1 µl each of restriction enzymes *Bam*HI (20000 U/µl) and *Hind*III (20000 U/µl), and the obtained fragment was run on agarose gel, and it was then separated and purified, so as to obtain a DNA fragment pCB cassette (31 ng/µl) consisting of approximately 2700 base pairs. Thereafter, a PCR product comprising the NPT gene cistron, which had been obtained by amplifying a DNA fragment by PCR with LA Taq DNA polymerase using primers having nucleotide sequences shown in SEQ ID NOS: 13 and 14, was cleaved with 1 µl each of restriction enzymes *Not*I (10000 U/µl) and *Nhe*I (5000 U/µl). Thereafter, the obtained fragment was run on agarose gel, and it was then separated and purified, so as to obtain a DNA fragment NEO cassette 2 (39 ng/µl) consisting of approximately 2700 base pairs. The pCB cassette was ligated to the NEO cassette 2, followed by transformation and the isolation of plasmids, so as to obtain pCB/GKT-Neo. Moreover, 3 µg of the pCB/GKT-Neo was cleaved with 1 µl each of restriction enzymes *Hind*III (20000 U/µl) and *Eco*RI (20000 U/µl), and the obtained fragment was run on agarose gel, and it was then separated and purified, so as to obtain a DNA fragment (CNB cassette; 11 ng/µl) consisting of approximately 1000 base pairs and comprising PCMV, an NPT gene and bGH-poly A.

Furthermore, 5 µg of the pND was cleaved with 1 µl each of restriction enzymes *Sac*II (20000 U/µl) and EcoRI (20000 U/µl) and 1.5 µl of a restriction enzyme *Nae*I (10000 U/µl). The obtained fragment was run on agarose gel, and it was then separated and purified, so as to obtain a DNA fragment (DHFR cassette 2; 33 ng/µl) consisting of approximately 2300 base pairs and comprising ColE1-Ori and a DHFR gene cistron having an SV40 early promoter from which an enhancer portion had been removed.

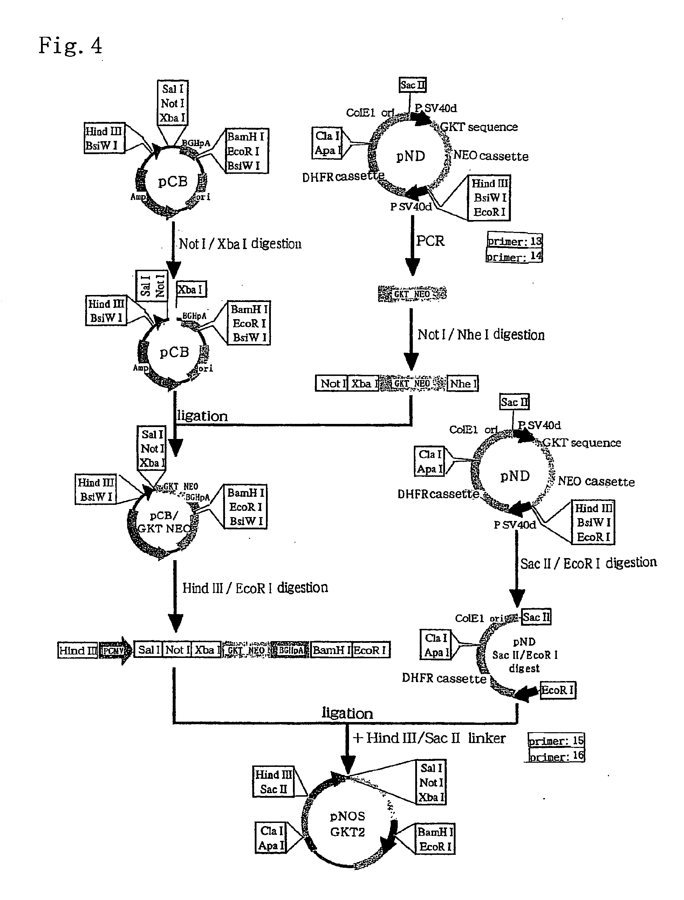

Still further, a linker (HS linker) having restriction enzyme-cleavage sites was obtained by annealing newly synthesized oligonucleotides having nucleotide sequences shown in SEQ ID NOS: 15 and 16. 1 µl of the CNB cassette, 1 µl of the DHFR cassette 2, and 1 µl of the HS linker were connected with one another by ligation, followed by transformation and the isolation of plasmids, so as to produce [pNOS-GKT2]. A flow chart for construction of a foreign gene expression vector [pNOS-GKT2] having an NPT gene cistron having no transcription promoters acting in mammalian cells is shown in Figure 4.

### (5) Production of foreign gene expression vector pNOS-GKT2B comprising bGH-poly A for termination of transcription located upstream of NPT gene cistron having no promoters

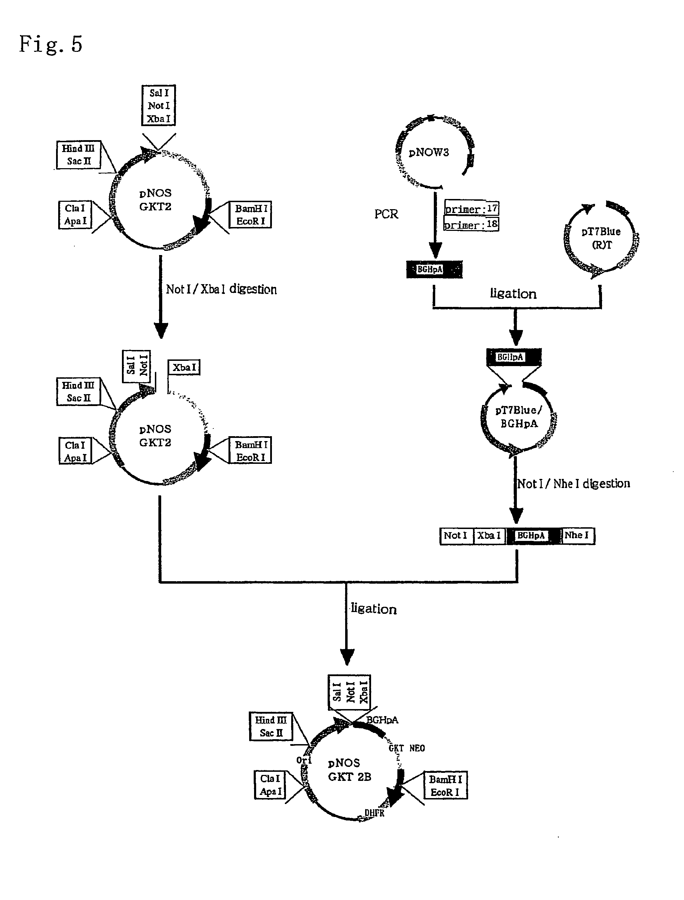

DNA was amplified from pNOW3 (Japanese Patent Laid-Open No. 10-179169) by PCR with LA Taq DNA polymerase, using primers having nucleotide sequences shown in SEQ ID NOS: 17 and 18. The thus amplified DNA was run on agarose gel, and it was then separated and purified, so as to obtain a DNA fragment (bGH-poly A; 42 ng/µl) consisting of approximately 200 base pairs. The DNA fragment was ligated to a plasmid pT7Blue(R), followed by transformation and the isolation of plasmids, so as to produce pT7Blue/BGHpA. 2.2 µg of the pT7Blue/BGHpA was cleaved with 1 µl each of restriction enzymes *Not*I (10000 U/µl) and *Nhe*I (5000 U/µl). The obtained fragment was run on agarose gel, and it was then separated and purified, so as to obtain a DNA fragment bGH-poly A cassette 2 (3 ng/µl) consisting of approximately 200 base pairs and comprising bGH-poly A. At the same time, 5 µg of the pNOS-GKT2 was cleaved with 1 µl each of restriction enzymes *Not*I (10000 U/µl) and *Xba*I (20000 U/µl). The obtained fragment was run on agarose gel, and it was then separated and purified, so as to obtain a DNA fragment pNOS-GKT2 cassette (13 ng/µl) consisting of approximately 4200 base pairs. 1 µl of the bGH-poly A cassette 2 was ligated to 1 µl of the pNOS-GKT2 cassette, followed by transformation and the isolation of plasmids, so as to produce pNOS-GKT2B. A flow chart for construction of a foreign gene expression vector pNOS-GKT2B comprising bGH-poly A for termination of transcription located upstream of an NPT gene cistron having no promoters is shown in Figure 5.

### (6) Production of foreign gene expression vector [pNOS-GKT2B-R] comprising rabbit β-globin gene intron for improvement of translation efficiency

DNA was amplified from rabbit genomic DNA (manufactured by CH Laboratories) by PCR with LA Taq DNA polymerase, using primers having nucleotide sequences shown in SEQ ID NOS: 19 and 20. The obtained PCR product (fragment length: approximately 600 base pairs), which corresponded to a portion (gloSA) encoding an intron comprising a rabbit β-globin gene splice donor and a splice acceptor sequence, was run on agarose gel, and it was then separated and purified. Thereafter, it was ligated to pT7Blue(R), followed by transformation and the isolation of plasmids, so as to prepare pT7B-gloSA.

5 µg of the pT7B-gloSA was cleaved with 1 µl each of restriction enzymes *Not*I (10000 U/µl) and *Xho*I (20000 U/µl). The obtained fragment was run on agarose gel, and it was then separated and purified, so as to obtain a DNA fragment (gloSA cassette; 22 ng/µl) consisting of approximately 600 base pairs and comprising gloSA. At the same time, 5 µg of the pNOS-GKT2B was cleaved with each of restriction enzymes *Not*I (10000 U/µl) and *Sal*I (20000 U/µl). The obtained fragment was run on agarose gel, and it was then separated and purified, so as to obtain a DNA fragment (pNOS-GKT2B cassette; 13 ng/µl) consisting of approximately 4400 base pairs. 1 µl of the gloSA cassette was ligated to 1 µl of the pNOS-GKT2B cassette, followed by transformation and the isolation of plasmids, so as to prepare pNOS-GKT2B-R.

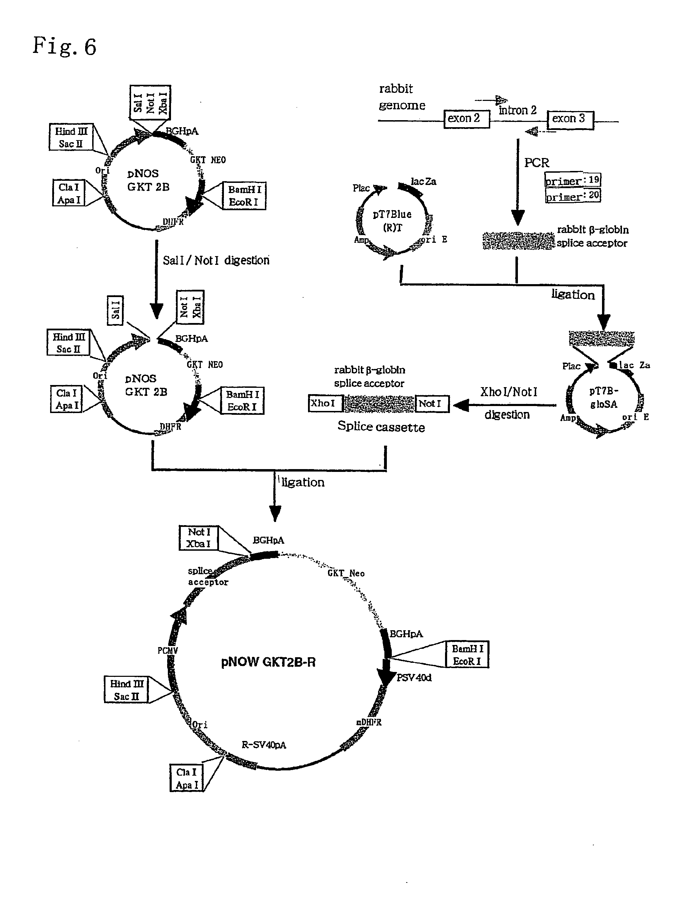

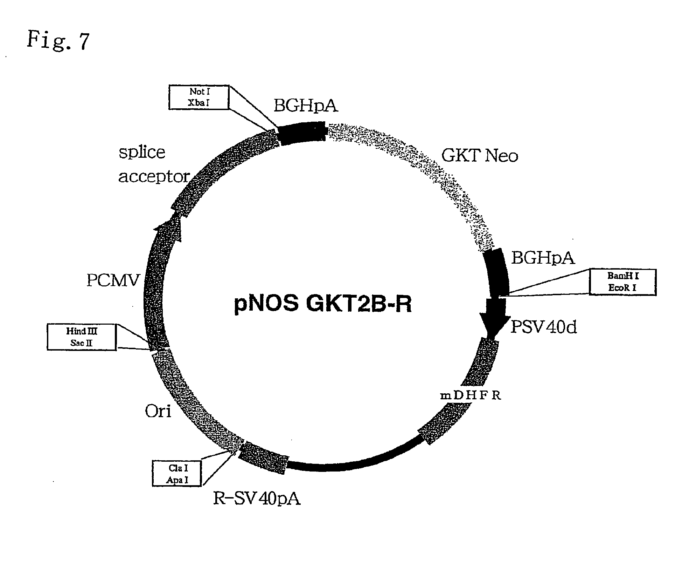

A flow chart for construction of a foreign gene expression vector [pNOS-GKT2B-R] comprising a rabbit β-globin gene intron for improvement of translation efficiency is shown in Figure 6. In addition, the structure of [pNOS-GKT2B-R] is shown in Figure 7, and the entire nucleotide sequence is shown in SEQ ID NO: 21.

### Example 2: Test of production of human granulocyte colony-stimulating factor using the expression vector of the present invention

One type of colony-stimulating factors, human granulocyte colony-stimulating factor (hG-CSF), was selected as a substance of interest, which was used in the gene transfer of the hG-CSF gene using an expression vector pNOS-GKT2B-R, and in the establishment of G418 resistant early clones. This protein has a sugar chain, and a normal form of recombinant protein cannot be obtained in the production using *Escherichia coli* or yeast as a host.

### (1) Production of hG-CSF expression vector [pNOS-GKT2B-R/hG-CSF]

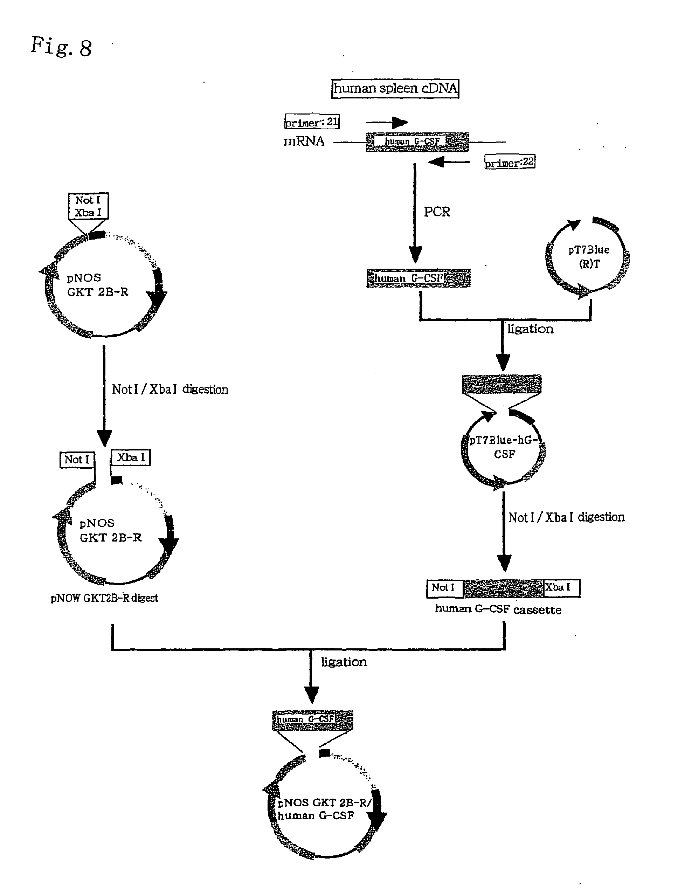

DNA was amplified from human spleen cDNA by PCR with LA Taq DNA polymerase, using primers having nucleotide sequences shown in SEQ ID NOS: 22 and 23. The thus amplified DNA was run on agarose gel, and it was then separated and purified, so as to obtain a fragment (hG-CSF fragment; 13 ng/µl) consisting of approximately 600 base pairs and comprising hG-CSF cDNA. This fragment was ligated to a plasmid pT7Blue(R), followed by transformation and the isolation of plasmids, so as to prepare pT7Blue/hG-CSF. The pT7Blue/hG-CSF was cleaved with 1 µl each of restriction enzymes *Not*I (10000 U/µl) and *Xba*I (20000 U/µl). The obtained fragment was run on agarose gel, and it was then separated and purified, so as to obtain a DNA fragment (hG-CSF cassette; 6 ng/µl) consisting of approximately 600 base pairs and comprising hG-CSF cDNA. At the same time, the pNOS-GKT2B-R was cleaved with 1 µl each of restriction enzymes *Not*I (10000 U/µl) and *Xba*I (20000 U/µl). The obtained fragment was run on agarose gel, and it was then separated and purified, so as to obtain a DNA fragment (pNOS-GKT2B-R cassette; 19 ng/µl) consisting of approximately 5000 base pairs. 1 µl of the hG-CSF cassette was ligated to 1 µl of the pNOS-GKT2B-R cassette, followed by transformation and the isolation of plasmids, so as to prepare pNOS-GKT2B. A flow chart for construction of an hG-CSF expression vector [pNOS-GKT2B-R/hG-CSF] is shown in Figure 8, and the entire nucleotide sequence is shown in SEQ ID NO: 24.

### (2) Introduction of hG-CSF expression vector [pNOS-GKT2B-R/hG-CSF] into mammalian cells, and selection of transformed strains using G418

1,500,000 DHFR-deficient CHO cells (DG44; obtained from Dr. Gail Urlaub; Somat. Cell Mol. Genet. 12, p. 555, 1986) placed in a 25cm² culture flask were transfected with 1 µg of pNOS-GKT2B-R/hG-CSF by the lipofectin method (using Effectene Transfection Reagent manufactured by Qiagen). The transfection was carried out according to an instruction book provided from the manufacturer. After a trypsin treatment for 48 hours, the cells were peeled off, and the number of the cells was counted. Thereafter, 300,000 cells were diluted with 100ml of 10% dialyzed fetal bovine serum-added Iscove's Modified Dalbecco's Medium containing 0.8 mg/ml G418. Thereafter, the dilution was dispersed in ten 96-well microtiter plates (960 wells) and then cultured in the presence of 5% carbon dioxide at 37°C for about 3 weeks. As a result, it was found that surviving cells (G418 resistant strains) were observed only in 117 wells. 61 G418 resistant clones were randomly picked up from the surviving cells. The clones were transferred to a 24-well plate together with 1 ml of 10% dialyzed fetal bovine serum-added Iscove's Modified Dalbecco's Medium containing 0.8 mg/ml G418, and they were then cultured until they became confluent. After the culture supernatant was discarded, 1 ml of PBS (manufactured by Invitrogen) was added to each well, and it was then discarded. Thereafter, 0.5 ml of a serum free medium for CHO cells, CHO-S-SFM II (manufactured by Invitrogen), was added thereto, followed by culture in the presence of 5% carbon dioxide at 37°C for 72 hours. Thereafter, the production amount of hG-CSF in the culture supernatant was analyzed.

### (3) Determination of the amount of hG-CSF produced by pNOS-GKT2B-R/hG-CSF transformed cell clones

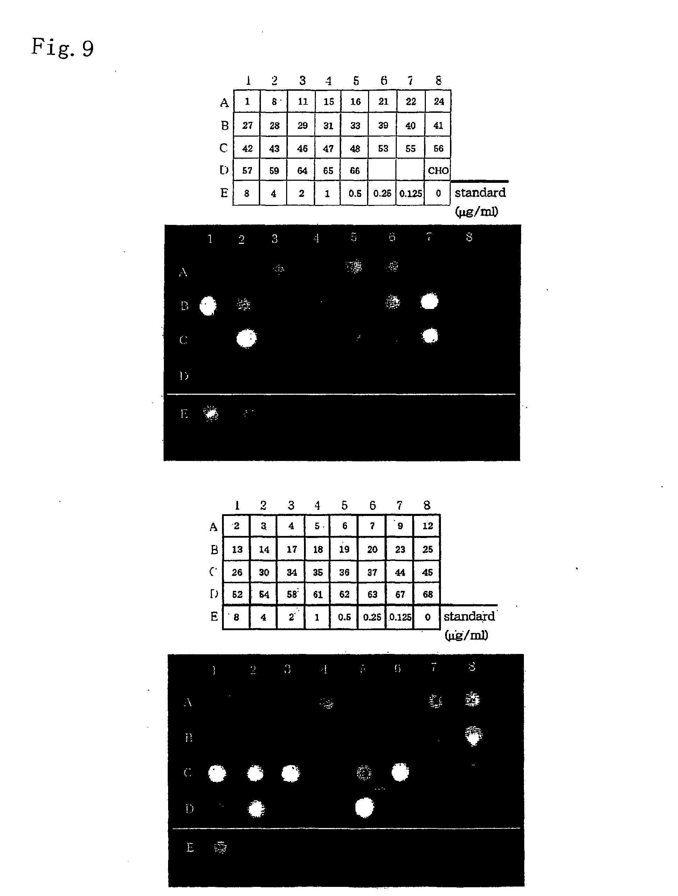

The production amount was determined by the dot blot method. 1 µl of a culture supernatant obtained by culturing for 72 hours, 1 µl of a commercially available recombinant hG-CSF (manufactured by PeproTech) serially diluted with a serum free medium for CHO cells CHO-S-SFM II (8 ~ 0.125 ng/µl), and 1 µl of CHO-S-SFM II were dotted on a nylon membrane (Nytran, 0.45 µm, manufactured by Schleicher & Schuell). It was air-dried for 1 hour and then subjected to blocking with Block Ace. Thereafter, it was reacted with an anti-hG-CSF antibody (1 µg/µl, manufactured by PeproTech) and then with a horseradish peroxidase (HRP)-labeled anti-rabbit IgG antibody (manufactured by DAKO). Using the reacted antibodies, the production amount of hG-CSF was determined by using a method of detecting the activity of HRP with a Super Signal West Pico reagent (manufactured by Pierce), applying LuminoCapture (manufactured by Ato). The results obtained by the dot blot method are shown in Figure 9.

### (4) Amount of hG-CSF produced by pNOS-GKT2B-R/hG-CSF transformed cell clones

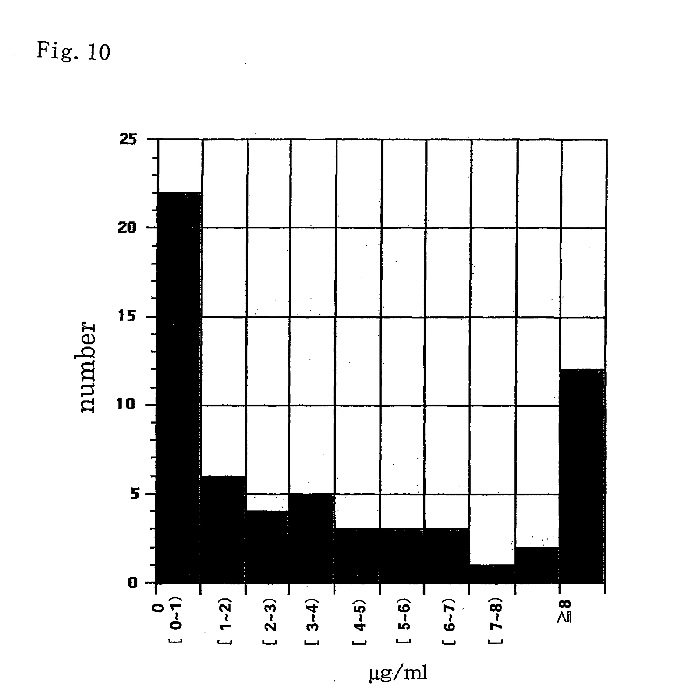

Figure 10 shows a distribution of the expression level of hG-CSF of each clone transfected with the expression vector pNOS-GKT2B of the present invention.

Approximately the two third (39 strains) out of sixty-one G418 resistant strains produced a significantly high level of hG-CSF (0.25 µg/µl or more). Moreover, 33 strains (54.1 %) out of the 61 strains produced hG-CSF at a level of 1 µg/µl or more. Further surprisingly, 12 strains (19.7%) out of the 61 strains produced hG-CSF at a level of 8 µg/µl or more. The highest production level was 178 µg/ml/3 days. When this value was compared with the data regarding early clones before gene amplification that had been obtained by representative expression vectors reported on publications or the like, it was found that this was the highest standard (DNA, 7, p. 651, 1988; Biotechnology, 10, p.1455, 1992; Biotechnology, 8, p. 662, 1990; Gene, 76, p. 19, 1989; Biotechnology, 9, p. 64, 1991).

Generally, it takes a period ranging from 6 months to 1 year to screen recombinant cells by gene amplification, and the results differ significantly depending on culture conditions or the concentration of a stimulant for amplification. Accordingly, it is considered to be adequate that primary performance comparison of expression vectors is carried out at the expression level of early clones before being amplified. It was found by such a comparison that the expression vector of the present invention has extremely high performance. As a result, it was confirmed that the "expression vector comprising a promoterless NPT gene cistron blocked by a strong polyadenylation signal" provides a very small number of G418 resistant strains, but enables the establishment of highly productive cell strains that produce proteins of interest with extremely high efficiency. From these results, it was proved that the expression vector of the present invention enables an extremely high level of protein expression.

### Example 3: Gene amplification of G418 resistant cell strains

Out of G418 resistant cell strains, clones producing hG-CSF at a level of 77 µg/ml/3 days were subcultured and then stabilized. Thereafter, gene amplification was carried out using MTX. First, the above clone was amplified in a medium containing 15 nM MTX for 2 weeks, in a medium containing 60 nM MTX for 2 weeks, then in a medium containing 250 nM MTX for 2 weeks, and then in a medium containing 1 µM MTX for further 2 weeks. As a result, the production level of hG-CSF was found to be increased to 117 µg/ml/3 days per medium. The concentration of MTX used in gene amplification is generally multi-step ranging between 10 nM and 10 µM, and 10 µM is often applied as the final concentration. However, considering toxicity to cells, exposure to a high concentration of MTX is problematic to establish stable recombinant cell strains. Accordingly, achievement of the high productivity in a low concentration of MTX is also an important evaluation standard. In the present invention, the concentration of MTX was set to 1 µM or lower. In addition, although the MTX-exposure period including selection is generally 6 to 12 months, it was set to about 8 weeks in the present experiment. In spite of these experimental conditions, an effective increase in the production amount of hG-CSF was observed, and it was confirmed that the vector of the present invention has high performance also in a gene amplification system.

### Example 4: Expression of recombinant human G-CSF by using pNOS

### (1) Production of pNOS/hG-CSF expression vector

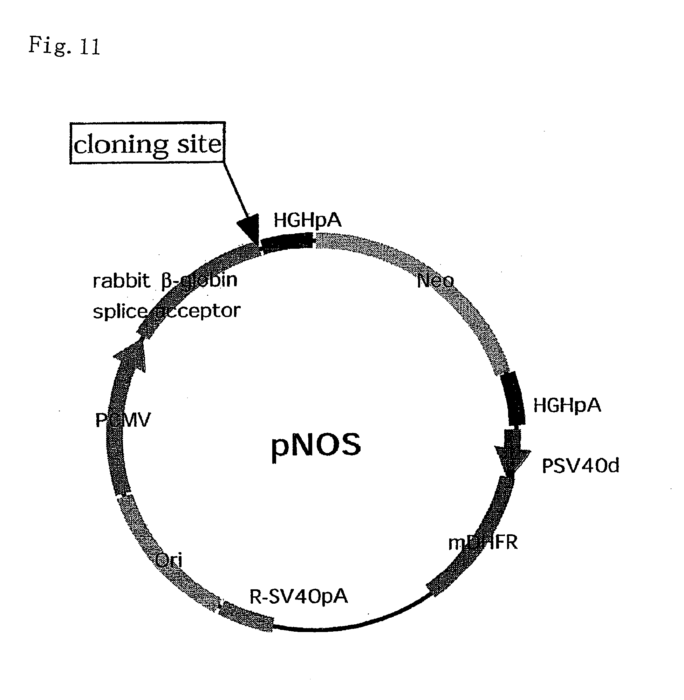

An expression vector pNOS was constructed in the same manner as in Example 1 with exceptions that Tn5 derived from *Escherichia coli* was directly used as an NEO resistant gene and that HGHpA was used instead of BGHpA. Moreover, according to the method described in Example 2 (1), a human granulocyte colony-stimulating factor (hG-CSF) gene was inserted into a cloning site, so as to construct an expression vector pNOS/hG-CSF. The structure of pNOS/hG-CSF is shown in Figure 11, and the entire nucleotide sequence is shown in SEQ ID NO: 25.

### (2) Measurement of expression level of recombinant human G-CSF obtained by using pNOS

When CHO cells that were being cultured in a 25 cm² flask were 40% to 80% confluent, a pNOS-human G-CSF expression vector was incorporated into the CHO cells using a transfection reagent commercially available from Qiagen. Two days later, the cells were treated with trypsin and then peeled off from the culture flask. Thereafter, the number of cells was counted using a blood cell counting chamber, and the cells were then dispersed in ten 96-well plates such that the concentration of the cells was set to 5 x 10⁵ cell/100 ml. Two weeks later, appearing colonies were counted, and as a result, 146 colonies were confirmed. 72 single colonies appearing in the ten 96-well plates were transferred to a 24-well plate. When the clones transferred to the 24-well plate became confluent, they were washed with 1 x PBS once. Thereafter, a serum free medium was added thereto, and after 3 days, the culture supernatant was collected.

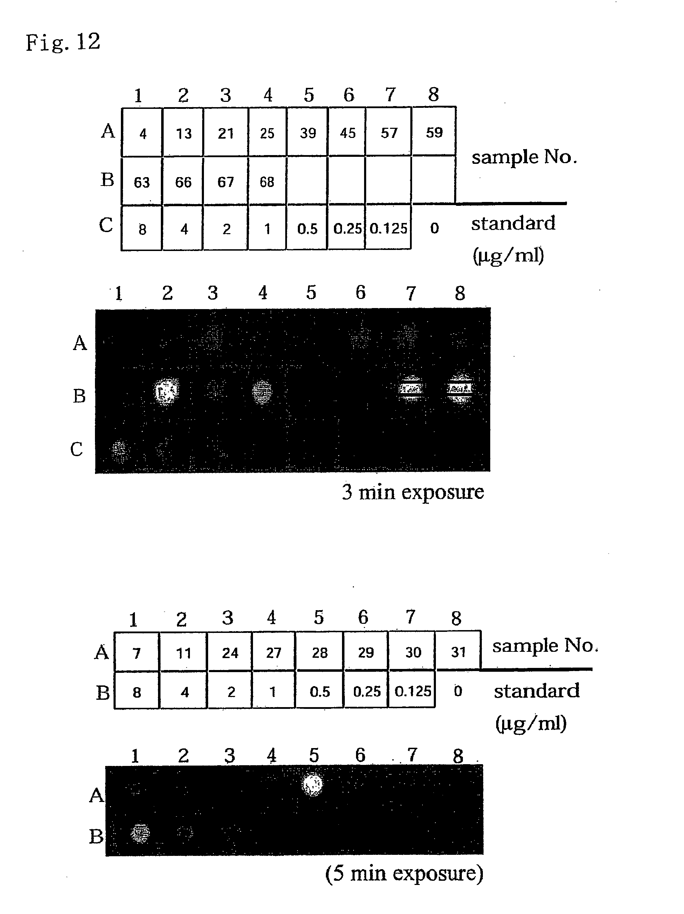

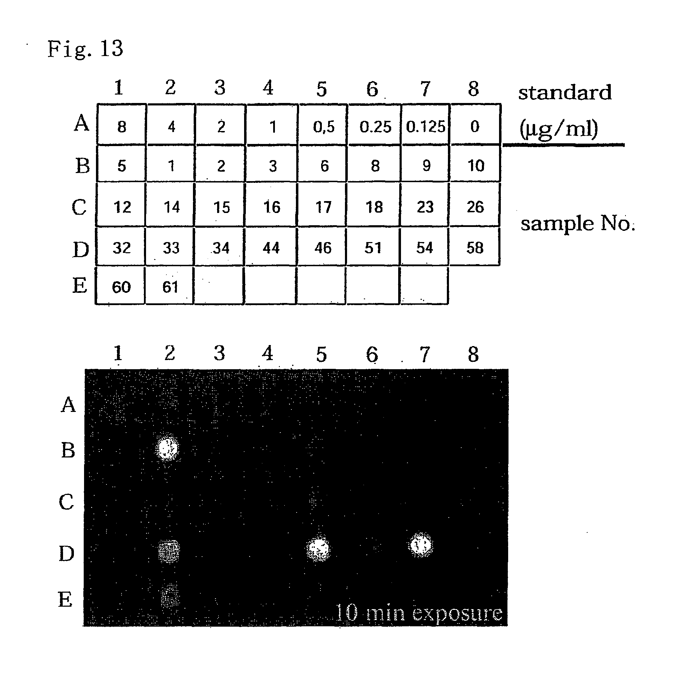

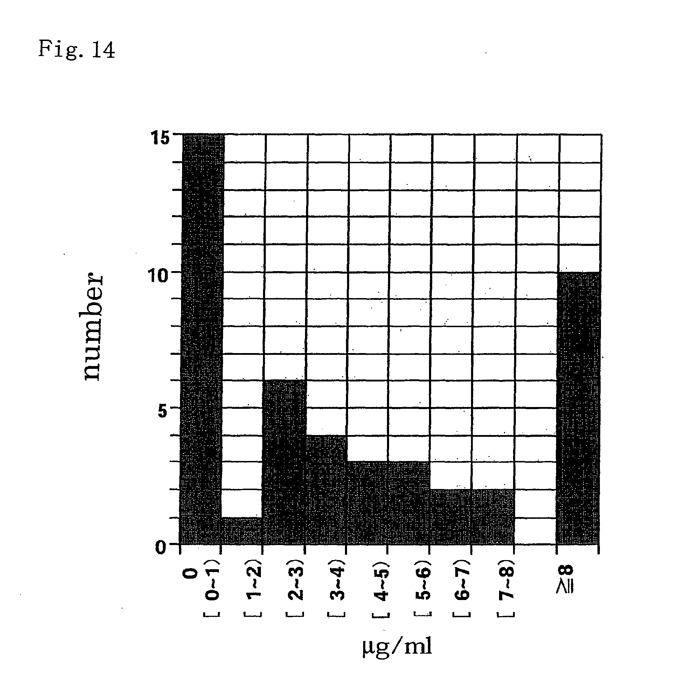

1 µl of the culture supernatant obtained after 3 days and 1 µl of a standard diluted line were spotted on a membrane. It was air-dried for 1 hour and then blocked. Thereafter, it was washed with TBS/T once. After completion of the washing, the primary antibody reaction was carried out using a 1 µg/ml rabbit-anti human G-CSF antibody. The reaction product was then washed with TBS/T three times. Thereafter, the secondary antibody reaction was carried out using HRP-anti-rabbit IgG. The reaction product was then washed with TBS/T three times. Thereafter, a chemiluminescence reaction was carried out using a Super Signal West Pico reagent, so as to confirm the expression level of human G-CSF. Examples of detecting hG-CSF by the dot blot method, which was expressed by the expression vector of the present invention pNOS/human G-CSF, are shown in Figures 12 and 13. In addition, a distribution of the expression level of hG-CSF of each clone, which was expressed by pNOS/human G-CSF, is shown in Figure 14.

The expression level of human G-CSF obtained by using a pNOS expression vector was determined by the dot blot method (the number of clones subjected to concentration test was 46). As a result, it was found that 65% or more clones produced hG-CSF at an expression level of 1 µg/ml or more by using pNOS, and that further 33% clones thereof produced hG-CSF at an expression level of 8 µg/ml or more.

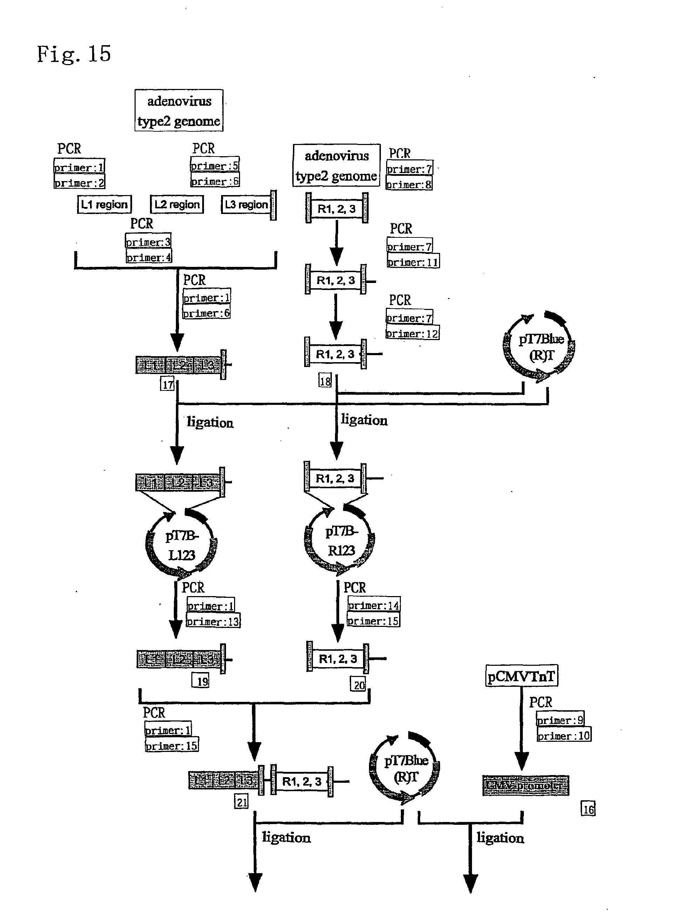

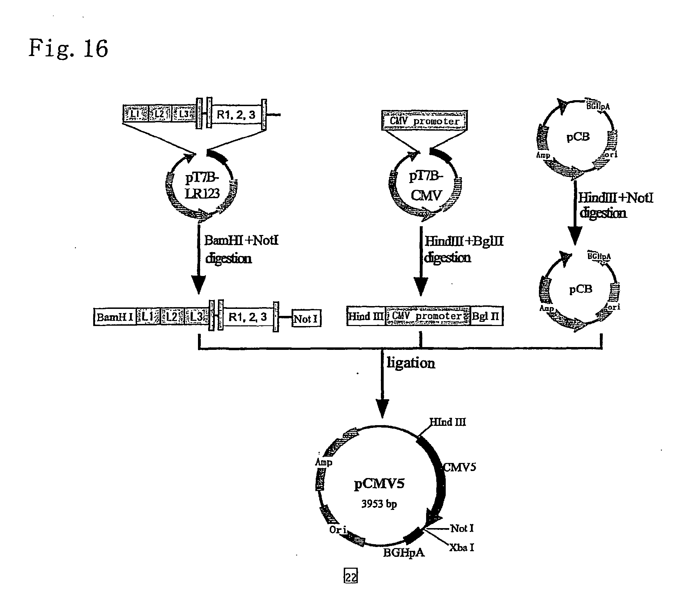

### Example 5: Preparation of CMV5 promoter (Figures 15 and 16)

(I-1) The L1 region was amplified by PCR from 1 µl of adenovirus type 2 genome. The PCR kit which was commercially available from TAKARA SHUZO CO., LTD was used, and the buffers and dNTP attached to the kit were used. First, 5 µl of 2.5 mM dNTP and 5 µl of 10 × Pyrobest PCR buffer were added to 36.5 µl of sterile distilled water, to which 1 µl of the sense primer [SEQ ID No. 26] (100 µM) and 1 µl of the antisense primer [SEQ ID No. 27] (100 µM) for the L1 region, and 1 µl of the template DNA were added. Then, 0.5 µl of a DNA polymerase Pyrobest (5 U/µl) was added to the resulting mixture. The mixture was completely mixed by pipetting, and PCR was carried out under the following conditions. After treatment under heating at 94 °C for 5 minutes, a cycle of three steps of 94 °C for 30 seconds, 55 °C for 30 seconds and 72 °C for 30 seconds was repeated 35 times. Finally, the mixture was treated at 72 °C for 5 minutes to complete the reaction. After the completion of PCR, the amplification of the gene region of interest was confirmed by electrophoresis using 1 % agarose gel. A band around 40 bp could be confirmed.
(I-2) The L2 region was amplified by PCR from 1 µl of adenovirus type 2 genome. The PCR kit which was commercially available from TAKARA SHUZO CO., LTD was used, and the buffers and dNTP attached to the kit were used. First, 5 µl of 2.5 mM dNTP and 5 µl of 10 × Pyrobest PCR buffer were added to 36.5 µl of sterile distilled water, to which 1 µl of the sense primer [SEQ ID No. 28] (100 µM) and 1 µl of the antisense primer [SEQ ID No. 29] (100 µM) for the L2 region, and 1 µl of the template DNA were added. Then, 0.5 µl of a DNA polymerase Pyrobest (5 U/µl) was added to the resulting mixture. The mixture was completely mixed by pipetting, and PCR was carried out under the following conditions. After treatment under heating at 94 °C for 5 minutes, a cycle of three steps of 94 °C for 30 seconds, 55 °C for 30 seconds and 72 °C for 30 seconds was repeated 35 times. Finally, the mixture was treated at 72 °C for 5 minutes to complete the reaction. After the completion of PCR, the amplification of the gene region of interest was confirmed by electrophoresis using 1 % agarose gel. A band around 70 bp could be confirmed.
(I-3) The L3 region was amplified by PCR from 1 µl of adenovirus type 2 genome. The PCR kit which was commercially available from TAKARA SHUZO CO., LTD was used, and the buffers and dNTP attached to the kit were used. First, 5 µl of 2.5 mM dNTP and 5 µl of 10 × Pyrobest PCR buffer were added to 36.5 µl of sterile distilled water, to which 1 µl of the sense primer [SEQ ID No. 30] (100 µM) and 1 µl of the antisense primer [SEQ ID No. 31] (100 µM) for the L3 region, and 1 µl of the template DNA were added. Then, 0.5 µl of a DNA polymerase Pyrobest (5 U/µl) was added to the resulting mixture. The mixture was completely mixed by pipetting, and PCR was carried out under the following conditions. After treatment under heating at 94 °C for 5 minutes, a cycle of three steps of 94 °C for 30 seconds, 55 °C for 30 seconds and 72 °C for 30 seconds was repeated 35 times. Finally, the mixture was treated at 72 °C for 5 minutes to complete the reaction. After the completion of PCR, the amplification of the gene region of interest was confirmed by electrophoresis using 1 % agarose gel. A band around 200 bp could be confirmed.
(I-4) The R1,2,3 region was amplified by PCR from 1 µl of adenovirus type 2 genome. The PCR kit which was commercially available from TAKARA SHUZO CO., LTD was used, and the buffers and dNTP attached to the kit were used. First, 5 µl of 2.5 mM dNTP and 5 µl of 10 × Pyrobest PCR buffer were added to 36.5 µl of sterile distilled water, to which 1 µl of the sense primer [SEQ ID No. 32] (100 µM) and 1 µl of the antisense primer [SEQ ID No. 33] (100 µM) for the R1,2,3 region, and 1 µl of the template DNA were added. Then, 0.5 µl of a DNA polymerase Pyrobest (5 U/µl) was added to the resulting mixture. The mixture was completely mixed by pipetting, and PCR was carried out under the following conditions. After treatment under heating at 98 °C for 1 minute, a cycle of three steps of 98 °C for 5 seconds, 55 °C for 20 seconds and 72 °C for 15 seconds was repeated 35 times. Finally, the mixture was treated at 72 °C for 2 minutes to complete the reaction. After the completion of PCR, the amplification of the gene region of interest was confirmed by electrophoresis using 1 % agarose gel. A band around 100 bp could be confirmed.
(I-5) The CMV promoter region was amplified by PCR using 1 µl of pCMVTnT (10 ng/µl) as template. The PCR kit which was commercially available from TAKARA SHUZO CO., LTD was used, and the buffer, dNTP and MgCl₂ attached to the kit were used. First, 5 µl of 2.5 mM dNTP, 5 µl of 25mM MgCl₂ and 5 µl of 10 × LA PCR buffer were added to 31.5 µl of sterile distilled water, to which 1 µl of the sense primer [SEQ ID No. 34] (100 µM) and 1 µl of the antisense primer [SEQ ID No. 35] (100 µM) for the CMV promoter, and 1 µl of the template DNA were added. Then, 0.5 µl of a DNA polymerase LA-Taq (5 U/µl) was added to the resulting mixture. The mixture was completely mixed by pipetting, and PCR was carried out under the following conditions. After treatment under heating at 94 °C for 5 minutes, a cycle of three steps of 94 °C for 30 seconds, 55 °C for 30 seconds and 72 °C for 30 seconds was repeated 35 times. Finally, the mixture was treated at 72 °C for 5 minutes to complete the reaction. After the completion of PCR, the amplification of the gene region of interest was confirmed by electrophoresis using 1 % agarose gel. A band around 650 bp could be confirmed (SEQ ID No.41).
(I-6) 5 µl of 3M NaoAc and 125 µl of 100 % ethanol were added to the PCR reaction solution in (I-1), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 40 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as L1 (the extract concentration = 82 ng/µl).
(I-7) 5 µl of 3M NaoAc and 125 µl of 100 % ethanol were added to the PCR reaction solution in (I-2), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 70 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as L2 (the extract concentration = 104 ng/µl).
(I-8) 5 µl of 3M NaoAc and 125 µl of 100 % ethanol were added to the PCR reaction solution in (I-3), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 200 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as L3 (the extract concentration = 88 ng/µl).
(I-9) 5 µl of 3M NaoAc and 125 µl of 100 % ethanol were added to the PCR reaction solution in (I-4), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 100 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as R123.
(I-10) 5 µl of 3M NaoAc and 125 µl of 100 % ethanol were added to the PCR reaction solution in (I-5), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 650 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as PCMV (the extract concentration = 54 ng/µl).
(I-11) The L123 region was amplified by PCR using 1 µl of each of the extracts of I-6, I-7 and I-8. The PCR kit which was commercially available from TAKARA SHUZO CO., LTD was used, and the buffers, dNTP and MgCl₂ attached to the kit were used. First, 5 µl of 2.5 mM dNTP, 5 µl of 25mM MgCl₂ and 5 µl of 10 × LA PCR buffer were added to 29.5 µl of sterile distilled water, to which 1 µl of the sense primer [SEQ ID No. 26] (100 µM) for the L 1 region and 1 µl of the antisense primer [SEQ ID No. 31] (100 µM) for the L3 region, and total 3 µl of the template DNAs were added. Then, 0.5 µl of a DNA polymerase LA-Taq (5 U/µl) was added to the resulting mixture. The mixture was completely mixed by pipetting, and PCR was carried out under the following conditions. After treatment under heating at 94 °C for 5 minutes, a cycle of three steps of 94 °C for 30 seconds, 55 °C for 30 seconds and 72 °C for 30 seconds was repeated 35 times. Finally, the mixture was treated at 72 °C for 5 minutes to complete the reaction. After the completion of PCR, the amplification of the gene region of interest was confirmed by electrophoresis using 1 % agarose gel. A band around 300 bp could be confirmed.
(I-12) 5 µl of 3M NaoAc and 125 µl of 100 % ethanol were added to the PCR reaction solution in (I-11), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 300 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as L123 (the extract concentration = 80 ng/µl) (SEQ ID No. 42].
(I-13) The R123' region was amplified by PCR using the extract of I-9. The PCR kit which was commercially available from TAKARA SHUZO CO., LTD was used, and the buffers and dNTP attached to the kit were used. First, 5 µl of 2.5 mM dNTP and 5 µl of 10 × Pyrobest PCR buffer were added to 36.5 µl of sterile distilled water, to which 1 µl of the sense primer [SEQ ID No. 32] (100 µM) and 1 µl of the antisense primer [SEQ ID No. 36] (100 µM) for the R123' region, and 1 µl of the template DNA were added. Then, 0.5 µl of a DNA polymerase Pyrobest (5 U/µl) was added to the resulting mixture. The mixture was completely mixed by pipetting, and PCR was carried out under the following conditions. After treatment under heating at 98 °C for 1 minute, a cycle of three steps of 98 °C for 5 seconds, 55 °C for 20 seconds and 72 °C for 15 seconds was repeated 35 times. Finally, the mixture was treated at 72 °C for 2 minutes to complete the reaction. After the completion of PCR, the amplification of the gene region of interest was confirmed by electrophoresis using 1 % agarose gel. A band around 150 bp could be confirmed.
(I-14) 5 µl of 3M NaoAc and 125 µl of 100 % ethanol were added to the PCR reaction solution in (I-13), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 150 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as R123-1 (the extract concentration = 107 ng/µl).
(I-15) The R123" region was amplified by PCR using the extract of I-14. The PCR kit which was commercially available from TAKARA SHUZO CO., LTD was used, and the buffers and dNTP attached to the kit were used. First, 5 µl of 2.5 mM dNTP and 5 µl of 10 × Pyrobest PCR buffer were added to 36.5 µl of sterile distilled water, to which 1 µl of the sense primer [SEQ ID No.32] (100 µM) and 1 µl of the antisense primer [SEQ ID No. 37] (100 µM) for the R123" region, and 1 µl of the template DNA were added. Then, 0.5 µl of a DNA polymerase Pyrobest (5 U/µl) was added to the resulting mixture. The mixture was completely mixed by pipetting, and PCR was carried out under the following conditions. After treatment under heating at 98 °C for 1 minute, a cycle of three steps of 98 °C for 5 seconds, 55 °C for 20 seconds and 72 °C for 15 seconds was repeated 35 times. Finally, the mixture was treated at 72 °C for 2 minutes to complete the reaction. After the completion of PCR, the amplification of the gene region of interest was confirmed by electrophoresis using 1 % agarose gel. A band around 150 bp could be confirmed.
(I-16) 5 µl of 3M NaoAc and 125 µl of 100 % ethanol were added to the PCR reaction solution in (I-15), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 150 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as R123-2 (the extract concentration = 95 ng/µl) (SEQ ID No. 43].
(I-17) 1 µl of the extract in I-12 as recovered by PCR (extract concentration expressed in ng/µl) and 1 µl of pT7Blue (at 50 ng/µl; Novagen) were mixed together. An equal volume of the ligation kit ver. 2 solution I (TAKARA SHUZO, Co., Ltd.) was added to the resulting mixture, and ligation reaction was carried out at 12 °C for 2 hours. Thereafter, transformants of Escherichia coli were obtained. After sequencing, then, a plasmid without any nucleotide substitution was selected. The plasmid thus recovered was defined as pT7B-L123.
(I-18) 1 µl of the extract in I-16 as recovered by PCR (extract concentration expressed in ng/µl) and 1 µl of pT7Blue (at 50 ng/µl; Novagen) were mixed together. An equal volume of the ligation kit ver. 2 solution I (TAKARA SHUZO, Co., Ltd.) was added to the resulting mixture, and ligation reaction was carried out at 12 °C for 2 hours. Thereafter, transformants of Escherichia coli were obtained. After sequencing, then, a plasmid without any nucleotide substitution was selected. The plasmid thus recovered was defined as pT7B-R123.
(I-19) 1 µl of the extract in I-10 as recovered by PCR (extract concentration expressed in ng/µl) and 1 µl of pT7Blue (at 50 ng/µl; Novagen) were mixed together. An equal volume of the ligation kit ver. 2 solution I (TAKARA SHUZO, Co., Ltd.) was added to the resulting mixture, and ligation reaction was carried out at 12 °C for 2 hours. Thereafter, transformants of Escherichia coli were obtained. After sequencing, then, a plasmid without any nucleotide substitution was selected. The plasmid thus recovered was defined as pT7B-CMV.
(I-20) The L123' region was amplified by PCR using pT7B-L123 (10 ng/µl) of I-17 as templete. The PCR kit which was commercially available from TAKARA SHUZO CO., LTD was used, and the buffers and dNTP attached to the kit were used. First, 5 µl of 2.5 mM dNTP and 5 µl of 10 × Pyrobest PCR buffer were added to 36.5 µl of sterile distilled water, to which 1 µl of the sense primer [SEQ ID No. 26] (100 µM) and 1 µl of the antisense primer [SEQ ID No. 38] (100 µM) for the L123' region, and 1 µl of the template DNA were added. Then, 0.5 µl of a DNA polymerase Pyrobest (5 U/µl) was added to the resulting mixture. The mixture was completely mixed by pipetting, and PCR was carried out under the following conditions. After treatment under heating at 94 °C for 5 minutes, a cycle of three steps of 95 °C for 30 seconds, 55 °C for 30 seconds and 72 °C for 30 seconds was repeated 35 times. Finally, the mixture was treated at 72 °C for 5 minutes to complete the reaction. After the completion of PCR, the amplification of the gene region of interest was confirmed by electrophoresis using 1 % agarose gel. A band around 300 bp could be confirmed.
(I-21) The R123-IgG3' region was amplified by PCR using pT7B-R123 (10 ng/µl) of I-18 as templete. The PCR kit which was commercially available from TAKARA SHUZO CO., LTD was used, and the buffers and dNTP attached to the kit were used. First, 5 µl of 2.5 mM dNTP and 5 µl of 10 × Pyrobest PCR buffer were added to 36.5 µl of sterile distilled water, to which 1 µl of the sense primer [SEQ ID No. 39] (100 µM) and 1 µl of the antisense primer [SEQ ID No. 40] (100 µM) for the R123 region, and 1 µl of the template DNA were added. Then, 0.5 µl of a DNA polymerase Pyrobest (5 U/µl) was added to the resulting mixture. The mixture was completely mixed by pipetting, and PCR was carried out under the following conditions. After treatment under heating at 94 °C for 5 minutes, a cycle of three steps of 95 °C for 30 seconds, 55 °C for 30 seconds and 72 °C for 30 seconds was repeated 35 times. Finally, the mixture was treated at 72 °C for 5 minutes to complete the reaction. After the completion of PCR, the amplification of the gene region of interest was confirmed by electrophoresis using 1 % agarose gel. A band around 200 bp could be confirmed.
(I-22) 5 µl of 3M NaoAc and 125 µl of 100 % ethanol were added to the PCR reaction solution in (I-20), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 300 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as L123-1 (the extract concentration = 87 ng/µl) (SEQ ID No. 44].
(I-23) 5 µl of 3M NaoAc and 125 µl of 100 % ethanol were added to the PCR reaction solution in (I-21), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 300 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as R123-IgG3 (the extract concentration = 79 ng/µl) [SEQ ID No. 45].
(I-24) The L123-R123-IgG3' region was amplified by PCR using 1 µl of each of the extract of I-22 and 23 as templetes. The PCR kit which was commercially available from TAKARA SHUZO CO., LTD was used, and the buffer, dNTP and MgCl₂ attached to the kit were used. First, 5 µl of 2.5 mM dNTP, 5 µl of 25mM MgCl₂ and 5 µl of 10 × LA PCR buffer were added to 29.5 µl of sterile distilled water, to which 1 µl of the sense primer [SEQ ID No. 1] (100 µM) for the L 1 region and 1 µl of the antisense primer [SEQ ID No. 15] (100 µM) for the R123-IgG3', and total 3 µl of the template DNA were added. Then, 0.5 µl of a DNA polymerase LA-Taq (5 U/µl) was added to the resulting mixture. The mixture was completely mixed by pipetting, and PCR was carried out under the following conditions. After treatment under heating at 98 °C for 1 minute, a cycle of three steps of 98 °C for 5 seconds, 55 °C for 20 seconds and 72 °C for 15 seconds was repeated 35 times. Finally, the mixture was treated at 72 °C for 2 minutes to complete the reaction. After the completion of PCR, the amplification of the gene region of interest was confirmed by electrophoresis using 1 % agarose gel. A band around 500 bp could be confirmed (SEQ ID No. 46].
(I-25) 5 µl of 3M NaoAc and 125 µl of 100 % ethanol were added to the PCR reaction solution in (I-24), and the mixture was centrifuged at 14,000 rpm for 10 minutes. After completion of the centrifugation, the pellet was confirmed, and the supernatant was gently discarded. 10 µl of 1 × DSB was added to the pellet to thoroughly dissolve the pellet. The total volume of the resulting solution was applied to 1 % agarose gel, and was subjected to electrophoresis at 50 mA for 25 minutes. By comparison with DNA markers, a band around 500 bp was confirmed and recovered, which was then frozen at -135 °C for 10 minutes and subsequently centrifuged at 14,000 rpm for 10 minutes. The supernatant from centrifugation was transferred to a fresh 1.5-ml Eppendorf tube. The resulting extract was defined as LR123 (the extract concentration = 56 ng/µl).
(I-26) 1 µl of the extract in I-25 as recovered by PCR (extract concentration expressed in ng/µl) and 1 µl of pT7Blue (at 50 ng/µl; Novagen) were mixed together. An equal volume of the ligation kit ver. 2 solution I (TAKARA SHUZO, Co., Ltd.) was added to the resulting mixture, and ligation reaction was carried out at 12 °C for 2 hours. Thereafter, transformants of Escherichia coli were obtained. After sequencing, then, a plasmid without any nucleotide substitution was selected. The plasmid thus recovered was defined as pT7B-LR123.
(I-27) 2.5 µg of the plasmid recovered in I-25 was used and digested with 1 µl of each of restriction enzymes BamHI (20,000 U/ml) and EcoRI (20,000 U/ml) (both from NEB) at 25 °C for overnight so as to cut out LR123. After treatment with the restriction enzymes, 1 µl of the resulting reaction solution was applied to 1 % agarose gel, and was subjected to electrophoresis at 50 mA for 25 minutes. Consequently, bands around 2800 bp and 400 bp were confirmed by comparison with DNA markers. Again, the total volume of the reaction solution was applied to 1 % agarose gel, and was subjected to electrophoresis at 50 mA for 25 minutes. A band confirmed around 400 bp was recovered. The gel extract recovered was frozen at -135 °C for 10 minutes. Subsequently, the product was centrifuged at 14,000 rpm for 10 minutes. The supernatant from centrifugation was transferred to a fresh 1.5-ml Eppendorf tube. The resulting extract was defined as LR123 digest (the extract concentration = 16 ng/µl).
(I-28) 2.5 µg of the plasmid recovered in I-19 was used and digested with 1 µl of each of restriction enzymes HindIII (20,000 U/ml) and BglII (10,000 U/ml) (both from NEB) at 25 °C for overnight so as to cut out the CMV promoter. After treatment with the restriction enzymes, 1 µl of the resulting reaction solution was applied to 1 % agarose gel, and was subjected to electrophoresis at 50 mA for 25 minutes. Consequently, bands around 2800 bp and 650 bp were confirmed by comparison with DNA markers. Again, the total volume of the reaction solution was applied to 1 % agarose gel, and was subjected to electrophoresis at 50 mA for 25 minutes. A band confirmed around 650 bp was recovered. The gel extract recovered was frozen at -135 °C for 10 minutes. Subsequently, the product was centrifuged at 14,000 rpm for 10 minutes. The supernatant from centrifugation was transferred to a fresh 1.5-ml Eppendorf tube. The resulting extract was defined as CMV promoter digest (the extract concentration = 14 ng/µl).
(I-29) 2.5 µg of pCB was used and digested with 1 µl of each of restriction enzymes HindIII (20,000 U/ml) and NotI (10,000 U/ml) (both from NEB) at 25 °C for overnight. After treatment with the restriction enzymes, total amount of the reaction solution was applied to 1 % agarose gel, and was subjected to electrophoresis at 50 mA for 25 minutes. Consequently, band around 3500 bp was confirmed by comparison with DNA markers, and was recovered. The gel extract recovered was frozen at -135 °C for 10 minutes. Subsequently, the product was centrifuged at 14,000 rpm for 10 minutes. The supernatant from centrifugation was transferred to a fresh 1.5-ml Eppendorf tube. The resulting extract was defined as pCB digest (the extract concentration = 30 ng/µl).
(I-30) 1 µl of the pCB digest (30 ng/µl) obtained in I-29, 1 µl of the LR123 digest (16 ng/µl) obtained in I-26 and 1 µl of the CMV promoter digest (14 ng/µl) obtained in I-27 were mixed together. An equal volume (3 µl) of the ligation kit ver. 2 solution I (TAKARA SHUZO, Co., Ltd.) was added to the resulting mixture, and ligation reaction was carried out at 16 °C for 30 minutes. Thereaftre, transformation of Escherichia coli was carried out to obtain a transformant. The plasmid recovered from the transformant was defined as pCMV5 [SEQ ID No. 47].

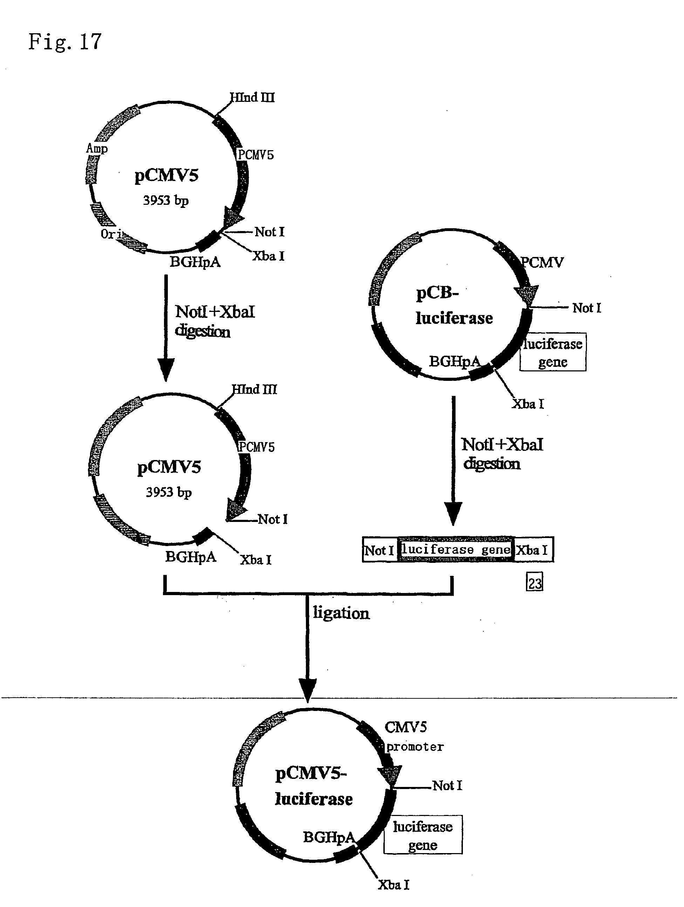

### Example 6: Preparation of pCMV5-luciferase (Figure 17)

(II-1) 2.5 µg of pCMV5 was used and digested with 1 µl of each of restriction enzymes NotI (10,000 U/ml) and XbaI (20,000 U/ml) (both from NEB) at 25 °C for overnight. After treatment with the restriction enzymes, total amount of the reaction solution was applied to 1 % agarose gel, and was subjected to electrophoresis at 50 mA for 25 minutes. Consequently, band around 4000 bp was confirmed by comparison with DNA markers, and was recovered. The gel extract recovered was frozen at -135 °C for 10 minutes. Subsequently, the product was centrifuged at 14,000 rpm for 10 minutes. The supernatant from centrifugation was transferred to a fresh 1.5-ml Eppendorf tube. The resulting extract was defined as pCMV5 digest (the extract concentration = 19 ng/µl).
(II-2) 2.5 µg of pCB-luciferase was used and digested with 1 µl of each of restriction enzymes NotI (10,000 U/ml) and XbaI (20,000 U/ml) (both from NEB) at 25 °C for overnight. After treatment with the restriction enzymes, 1 µl of the resulting reaction solution was applied to 1 % agarose gel, and was subjected to electrophoresis at 50 mA for 25 minutes. Consequently, bands around 3500 bp and 1600 bp were confirmed by comparison with DNA markers. Again, the total volume of the reaction solution was applied to 1 % agarose gel, and was subjected to electrophoresis at 50 mA for 25 minutes. A band confirmed around 1600 bp was recovered. The gel extract recovered was frozen at -135 °C for 10 minutes. Subsequently, the product was centrifuged at 14,000 rpm for 10 minutes. The supernatant from centrifugation was transferred to a fresh 1.5-ml Eppendorf tube. The resulting extract was defined as luciferase gene digest (the extract concentration = 27 ng/µl) (SEQ ID NO.48).
(II-3) 1 µl of the pCMV5 digest (19 ng/µl) ontained in (II-1) and 1 µl of the luciferase gene digest (27 ng/µl) obtained in (II-2) were mixed together. An equal volume (2 µl) of the ligation kit ver. 2 solution I (TAKARA SHUZO, Co., Ltd.) was added to the resulting mixture, and ligation reaction was carried out at 16 °C for 30 minutes. Thereafter, transformation of Escherichia coli was carried out to obtain a transformant of Escherichia coli. The plasmid thus recovered from the transformant was defined as pCMV5-luciferase.

### Example 7: Assay of the activity of the CMV5 promoter by luciferase assay

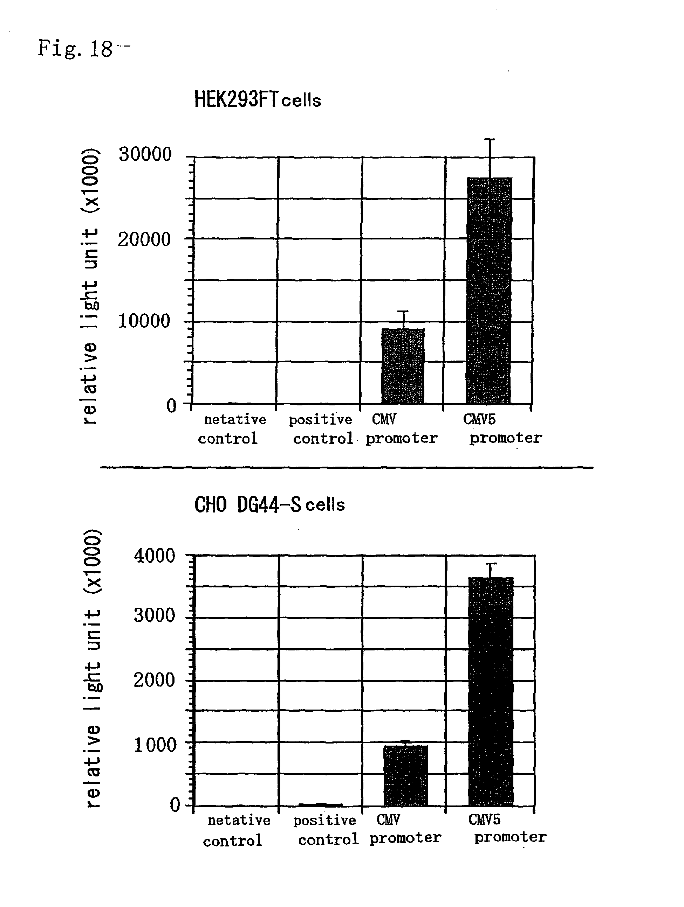

(III-1) In order to assay the activity of the CMV5 promoter with CHO DG44-S and HEK293RT, these cells were respectively cultured in 25-cm² flasks. The cells cultured in the 25-cm² flask were centrifuged in a 15-ml centrifuge tube on the day of transfection. After centrifugation, the culture supernatant was discarded. To one of the resulting centrifuge tubes was added 5 ml of a CHO-S-SFM II culture medium (GIBCO) containing 100 mM hypoxanthine and 10 mM thymidine. To the other centrifuge tube was added 5 ml of a Free Style medium (GIBCO). The cells were counted in both of the centrifuge tubes and then individually transferred to a 24-well plate to 2.5 × 10⁵ cells/well.
(III-2) 0.35 ml of each of the culture media was added to 61 µl of a transfection reagent (QIAGEN) containing 166 ng of the DNA (pCMV5-luciferase) per well, and the mixture was added in the 24-well plates, and the culturing was carried out for 48 hours.
(III-3) 48 hours later, the cells were collected in a 1.5-ml tube, and the supernatants were discarded. 100 µl of PLD-30 (the reagent attached to the Picker Gene Dual sea pansy kit; manufactured by Toyo Ink Group Co., Ltd.) was added to the resulting cells. Then, the resulting mixture was gently agitated and left to stand for 15 minutes. 15 minutes later, the tube was centrifuged at 200 × g for 10 minutes. 5 µl of the supernatant recovered by centrifugation was added to 195 µl of the reagent PLD-30, and luciferase assay was carried out using a fluorescence assay apparatus (ATTO; Luminescencer PSN R). Thus, an activity which is stronger than that of the CMV promoter could be confirmed. The results are shown in Figure 18.

### INDUSTRIAL APPLICABILITY

The present invention provides an expression vector capable of high-level production of genetically recombinant proteins using an animal cell as a host.

## Claims

1. An expression vector inducing high productivity of genetically recombinant proteins in animal host cells, which comprises a highly expression-inducible promoter, a multicloning site for insertion of gene and a polyadenylation signal sequence in this order from upstream, and further comprises downstream thereof a drug resistant gene that does not have a promoter capable of acting in animal cells.

2. The expression vector according to claim 1, wherein the highly expression-inducible promoter is a human cytomegalovirus Major Immediately-Early antigen promoter, CMV5 promoter (a synthetic chimeric promoter), a β-actin promoter, or an SV40 early promoter.

3. The expression vector according to claim 1 or 2, wherein the drug resistant gene is a neomycine resistant gene, codon-disoptimized neomycine resistant gene, a hygromycin resistant gene, a zeocin resistant gene, or a blasticidin resistant gene.

4. The expression vector according to any one of claim 1 to 3, wherein the drug resistant gene is a neomycine phosphotransferase gene.

5. The expression vector according to any one of claims 1 to 4, wherein the drug resistant gene is a neomycine phosphotransferase gene derived from *Escherichia coli* transposon Tn5.

6. The expression vector according to any one of claims 1 to 5, wherein a trace amount of the messenger RNA or protein of a drug resistant gene is generated by a read-through effect which is achieved by the fact that the drug resistant gene exists downstream of the highly expression-inducible promoter blocked by the polyadenylation signal sequence.

7. The expression vector according to any one of claim 1 to 6, which further comprises, downstream of the drug resistant gene, a dihydrofolate reductase gene or codon-disoptimized dihydrofolate reductase gene that does not have a promoter capable of acting in animal cells.

8. The expression vector according to any one of claims 1 to 7, which further comprises, downstream of the drug resistant gene, a highly expression-inducible promoter, a multicloning site for insertion of gene and a polyadenylation signal sequence in this order from upstream, and further comprises downstream thereof a dihydrofolate reductase gene or codon-disoptimized dihydrofolate reductase gene that does not have a promoter capable of acting in animal cells.

9. The expression vector according to any one of claims 1 to 6, which comprises a dihydrofolate reductase gene, or codon-disoptimized dihydrofolate reductase gene ligated downstream of a promoter with low expression inductivity.

10. The expression vector according to any one of claims 7 to 9, wherein the promoter with low expression inductivity is a promoter derived from a gene of a protein that is generally expressed in trace amounts in mammalian cells, a promoter derived from a viral antigen gene that hardly ever infects mammals, or a promoter obtained by removing an enhancer sequence from the above promoters.

11. A recombinant expression vector which is obtained by incorporating a gene of interest into the multicloning site for insertion of gene of the expression vector according to any one of claims 1 to 6.

12. A recombinant vector which is obtained by incorporating a gene of interest into the multicloning site for insertion of gene of the expression vector according to any one of claims 7 to 10.

13. A transformant comprising the expression vector according to any one of claims 1 to 10 or the recombinant expression vector according to claim 11 or 12.

14. A method for producing a transformant that highly expresses a gene of interest, which comprises introducing the recombinant expression vector according to claims 11 or 12 into animal host cells.

15. A method for obtaining a transformant capable of stably producing proteins in a serum free medium, which comprises introducing the recombinant expression vector according to claim 11 or 12 into animal host cells so as to obtain a transformant, and acclimating the obtained transformant to the serum free medium.

16. A method for amplifying a gene of interest, which comprises introducing the recombinant expression vector according to claim 12 into a dihydrofolate reductase-deficient CHO cell, and culturing the cell in the presence of methotrexate.
